# EUROPEAN PATENT SPECIFICATION

(11) **EP 1 644 474 B1**
(45) Date of publication and mention of the grant of the patent: **27.06.2012**
(21) Application number: 04748728.5
(22) Date of filing: 12.07.2004
(51) Int. Cl.: C12N 1/00

(54) **COMPOSITIONS AND METHODS FOR STABLE ISOTOPE LABELLING OF BIOLOGICAL COMPOUNDS**
ZUSAMMENSETZUNGEN UND VERFAHREN ZUR STABILEN ISOTOPMARKIERUNG BIOLOGISCHER VERBINDUNGEN
COMPOSITIONS ET PROCEDES DE MARQUAGE DE COMPOSES BIOLOGIQUES AVEC DES ISOTOPES STABLES

(30) Priority: 11.07.2003 WO PCT/NL03/00514
(43) Date of publication of application: 12.04.2006
(73) Proprietor: Egorova-Zachernyuk, Tatiana A., 2332 BD Leiden (NL)
(72) Inventor: Egorova-Zachernyuk, Tatiana A., 2332 BD Leiden (NL)
(74) Representative: van Westenbrugge, Andries
(86) International application number: PCT/NL2004/000503
(87) International publication number: WO 2005/005616

(56) References cited:
- WO-A-94/18339
- HANSEN ANDREW P ET AL: "A practical method for uniform isotopic labeling of recombinant proteins in mammalian cells" BIOCHEMISTRY, vol. 31, no. 51, 1992, pages 12713-12718, XP002264470 ISSN: 0006-2960 cited in the application
- LUSTBADER J W ET AL: "Expression of human chorionic gonadotropin uniformly labeled with NMR isotopes in Chinese hamster ovary cells: an advance toward rapid determination of glycoprotein structures." JOURNAL OF BIOMOLECULAR NMR. JUN 1996, vol. 7, no. 4, June 1996 (1996-06), pages 295-304, XP009045443 ISSN: 0925-2738 cited in the application
- OPELLA S J ET AL: "Structures of the M2 channel-lining segments from nicotinic acetylcholine and NMDA receptors by NMR spectroscopy" NATURE STRUCTURAL BIOLOGY, vol. 6, no. 4, April 1999 (1999-04), pages 374-379, XP001155776 ISSN: 1072-8368
- ARCHER S J ET AL: "Transforming growth factor beta-1: NMR signal assignments of the recombinant protein expressed and isotopically enriched using chinese hamster ovary cells" BIOCHEMISTRY, AMERICAN CHEMICAL SOCIETY. EASTON, PA, US, vol. 32, no. 4, 1993, pages 1152-1163, XP002243159 ISSN: 0006-2960
- TROPIS MARIELLE ET AL: "Composition and phase behaviour of polar lipids isolated from Spirulina maxima cells grown in a perdeuterated medium" BIOCHIMICA ET BIOPHYSICA ACTA, vol. 1284, no. 2, 1996, pages 196-202, XP002264471 ISSN: 0006-3002
- EGOROVA-ZACHERNYUK T A ET AL: "HETERONUCLEAR 2D-CORRELATIONS IN A UNIFORMLY [13C,15N] LABELED MEMBRANE-PROTEIN COMPLEX AT ULTRA-HIGH MAGNETIC FIELDS" JOURNAL OF BIOMOLECULAR NMR, ESCOM, LEIDEN, NL, vol. 19, no. 3, March 2001 (2001-03), pages 243-253, XP009019446 ISSN: 0925-2738
- WOOD MATTHEW J ET AL: "Production of large quantities of isotopically labeled protein in Pichia pastoris by fermentation" JOURNAL OF BIOMOLECULAR NMR, vol. 13, no. 2, February 1999 (1999-02), pages 149-159, XP009019488 ISSN: 0925-2738

## Description

### Field of the invention

The present invention is concerned the labelling of biological compounds with stable isotopes. In the methods of the invention microorganisms are grown on mineral media comprising carbon and nitrogen sources that contain stable isotopes to produce biomass that is uniformly labelled with the stable isotopes. The biomass is extracted with organic solvent to produce lipids. The rest of the delipidised biomass is then hydrolysed to produce labelled amino acids and other nutrients, which are used to prepare a culture medium for a host cell of choice for the production of biological compounds that are uniformly labelled with stable isotopes. Uniform labelling with stable isotopes allows the determination of the three-dimensional structure by NMR spectroscopy of the biological compound. The biological compound preferably is a biological macromolecule, such as e.g. a mammalian transmembrane protein.

### Background of the invention

The concept of "rational drug design" requires detailed knowledge of the three-dimensional (3-D) structure of biological macromolecules, particularly proteins. Rational drug design involves the determination of the 3-D structure of an "active part" of a particular biological molecule that is suspected of being involved in the etiology of a disease. The biological molecule may e.g. be a receptor, an enzyme, a hormone, or any other biologically active molecule. Once the 3-D structure of biological molecule, or at least its active site, is known, scientists can use computer modelling to design molecules that will block, mimic or enhance the natural biological activity of the molecule. Knowledge of the 3-D structure of biological molecules is therefore of great practical and commercial significance.

The first technique available for the determination of 3-D structures of proteins and other large biomolecules was X-ray diffraction. The structures of hemoglobin and DNA were among the first to be determined using this technique. X-ray diffraction, however, requires that the molecule to be investigated is available in the form of a crystal because the 3-D structure is calculated from a pattern of X-rays that are refracted by the atoms of the ordered molecules in the crystal. Thereby the use of X-ray diffraction for 3-D determination is limited to molecules that can be crystallised. As a consequence the vast majority of membrane proteins cannot be subjected to X-ray diffraction but also attempts at crystallisation of many soluble proteins have failed as crystallisation is more an empirical art rather than science. At present, the protein database holds only 100 entries (0.7%) integral membrane domains analysed by X-ray crystallographically (of which eleven are no more than isolated single α-helical transmembrane domains), in contrast to 13600 entries for soluble proteins. The most recently applied techniques and strategies for structural genomics are discussed in detail by Essen (2002, Gene Funct. Dis. 3: 39; see also Jack, 2002 DDT 7: 35).

More recently, another technique, nuclear magnetic resonance ("NMR") spectroscopy, has been developed to determine the 3-D structures of biological molecules, and particularly proteins. NMR spectroscopy does not require crystallisation of the molecule to be analysed and should thus be amenable to in principal any biomolecule of interest, including transmembrane proteins and other molecules that are refractory to crystallisation. A further advantage of NMR spectroscopy is its ability to provide a detailed picture of the dynamics of the interaction of proteins with their ligands (Roberts, G.C.K, (2000) DDT. 5: 230).

NMR spectroscopy involves placing the molecule to be analysed (usually in a suitable solvent) in a powerful magnetic field and irradiating it with a strong radio signal. The nuclei of the various atoms will align themselves with the magnetic field until energised by the radio signal. They then absorb this energy and re-radiate (resonate) it at a frequency dependent on i) the type of nucleus and ii) the chemical environment as largely determined by bonding of the nucleus. Moreover, resonances can be transmitted from one nucleus to another, either through bonds or through 3-D space, thus giving information about the environment of a particular nucleus and nuclei in the vicinity of it.

However, not all nuclei are NMR active, in particular, not all isotopes of the same element are active. The most commonly used stable isotopes for macromolecular NMR are ¹³C, ¹⁵N, and ²H. There is also natural NMR active nuclei such as ³¹P, ¹⁹F, and ¹H. For larger molecules, such as proteins, a sufficiently strong signal in NMR spectra requires enrichment with NMR active stable isotopes. Depending on the NMR-probe used, e.g. "ordinary" hydrogen, ¹H, is NMR active, whereas heavy hydrogen (deuterium), i.e. ²H, is not. Thus, any hydrogen-containing molecule can be rendered "invisible" in the hydrogen NMR spectrum by replacing all the ¹H hydrogen atoms with ²H. For this reason NMR spectra of water-soluble materials are determined in solution in ²H₂O (heavy water), so as to avoid the water signal. Conversely, "ordinary" carbon, ¹²C is NMR inactive whereas the stable isotope ¹³C (about 1% of total carbon in nature) is active. Similarly, "ordinary" nitrogen, ¹⁴N, is NMR inactive whereas the stable isotope ¹⁵N, (again about 1% of total nitrogen in nature) is active. The most commonly used stable isotopes for biomolecular NMR are therefore ¹³C, ¹⁵N, and ²H.

For small molecules, i.e. with a molecular weight below about 1000 Dalton, the low natural levels of NMR active isotopes were found to be sufficient to generate the required signal in NMR spectra. However, for larger molecules, such as proteins, a sufficiently strong signal in NMR spectra requires enrichment with NMR active stable isotopes. Isotopic enrichment results in increased sensitivity and resolution, and in reduced complexity of the NMR spectra. Isotopic enrichment has allowed the efficient use of heteronuclear multidimensional NMR experiments and has provided alternative approaches to the spectral assignment process and further structural constrains from spin-spin coupling.

A method of achieving isotopic enrichment or substitution in biomolecules was to grow microorganisms capable of producing the proteins in growth media labelled with these isotopes. To this end, a bacterium or yeast, transformed by genetic engineering to produce a particular protein of choice, e.g. a mammalian protein of medical importance, have been grown in a medium containing ¹³C and/or ¹⁵N labelled substrates. In practice, these media usually consist of ¹³C-labelled glucose and/or ¹⁵N-labelled ammonium salts (see e.g. Kay et al., 1990, Science, 249: 411, and references herein). WO 90/15525 discloses bacterial and yeast nutrient media containing labelled protein hydrolysates to improve growth and protein production.

This approach is, however, not satisfactory for most proteins of interest in rational human drug design which by definition are mammalian and preferably human in origin. Many mammalian proteins that are expressed in microorganisms such as bacteria or yeasts are not produced in a form that is identical to or that would even resemble the native protein. This is particularly true for mammalian membrane proteins and secreted proteins both of which may undergo significant post-translational modifications including appropriate folding, cross-linking of inter- and intra-molecular chains through disulphide bridges, glycosylation, acylation, phosphorylation and other chemical modifications, and proteolytic cleavage into active forms. Many of these modifications cannot reliably be effected by bacterial and yeast host cells. As a consequence, bacterial or yeast-produced proteins cannot be used for structure function studies because they don't have or even resemble the relevant native structure. Frequently, they do not possess the biological activity of the native protein and, in some cases, mammalian proteins cannot be produced in bacteria at all. For these reasons host-vector systems utilising both mammalian cells and insect cells have been developed. Mammalian cell lines, such as Chinese hamster ovary (CHO) cells, COS cells and insect-cell lines, such as the *Spodoptera frugiperda* cell lines Sf9 and Sf21 (Luckow and Summers, 1988, Biotechnol. 6: 47-55), have been found to produce recombinant mammalian proteins with post-translational modifications similar to those of the natural protein.

However, a problem of using mammalian or insect cells for labelling of proteins with stable isotopes such as ¹³C or ¹⁵N, is that whereas bacteria can grow on a simple mineral media, mammalian and insect cells require complex mixtures of nutrients, including at least amino acids. This problem has been addressed in the art by using hydrolysates of protein-containing labelled biomass as a source for labelled amino acids. Preferably, for production of labelled biomass microorganisms such as green or blue-green algae are used that can grow solely on simple (and therefore cheap) labelled carbon and nitrogen sources, such as ¹⁵NH₃, ¹⁵NO₃⁻, ¹⁵NO₂⁻, H¹³CO₃⁻, ¹⁵N₂, ¹³CO₂ or ²H₂O, thereby incorporating the labelled atoms via photosynthesis into complex biomolecules such as proteins, carbohydrates, lipids and nucleic acids. A practical method for uniform isotopic labelling of recombinant proteins in mammalian cells is described by Hansen et al. (1992, Biochem. 31: 12713). These authors disclose hydrolysis of isotopically labelled algal and bacterial proteins for the production of isotopically labelled proteins from mammalian cells for NMR structural studies. Hydrolysis is performed with methanesulphonic acid in the presence of tryptamine and imidazole and the amino acids were then purified by ion-exchange chromatography. However, the hydrolysis conditions employed destroy asparagine, glutamine and cysteine residues and leave just a trace of tryptophane. The mammalian culture medium therefore needed to be supplemented with commercially available cysteine and enzymatically synthesised glutamine which was only labelled at N and C atoms. The medium was further supplemented with 5% of heat-treated serum and thus contained non-labelled substances that could be used for protein synthesis.

WO 94/18339 discloses nutrient media for labelling proteins expressed in mammalian and insect cells. The nutrient media are composed of labelled amino acids purified from hydrolysed biomass of the green algea *Chlorella,* and supplemented with the synthetic labelled amino acids cysteine, glutamine and asparagine and with labelled glucose and pyruvate. Similar techniques for producing isotopically labelled proteins and macromolecules, such as glycoproteins, in mammalian or insect cells have been described in e.g. U.S. Pat. No.'s. 5,393,669 and 5,627,044, Weller (1996, Biochem., 35: 8815-23) and Lustbader (1996, J. BiomoL NMR 7: 295-304).

WO 99/11589 discloses methods for labelling proteins that are isotopically labelled in the backbone structure, but not in the amino acid side chains. The proteins are produced in cell culture media that are composed of backbone labelled amino acids that are chemically synthesised from the doubly protected glycine derivative amino acids.

For the provision of a nutrient medium for producing labelled proteins in insect or mammalian cells, the art thus far has focussed mostly on obtaining isotopically labelled amino acids from hydrolysed biomass. Amino acids that were not present in sufficient quantities in hydrolysates as well as other nutrients such as glucose or pyruvate were simply obtained commercially to supplement the media. Despite the long felt need in the art for NMR structural studies on mammalian (transmembrane) proteins, and despite the fact that such nutrient media have been disclosed since 1994, no reports have since issued of NMR structural studies on isotopically labelled proteins produced from mammalian or insect cells grown in these media. It is thus object of the invention to improve culture media for growing insect or mammalian cells for the production of isotopically labelled proteins for NMR analysis.

### Description of the invention

In a first aspect the invention relates to a method for producing a nutrient medium for growing mammalian or insect cells in culture. In the nutrient medium, for at least one of H, C or N, 95% or more of the atoms in substrates that are used by the cells for synthesis of biomolecules, are isotopically labelled. The method preferably comprises the steps of: (a) growing an organism on a mineral medium which supports growth of the organism, whereby in the medium substantially all of the assimilable atoms, for at least one of H, C or N, are isotopically labelled, to produce labelled biomass; (b) autolysing the biomass of an organism grown as in (a) to produce an autolysate; and, (c) composing the nutrient medium by combining the autolysate as obtained in (b) with further components necessary for growth of the mammalian or insect cells. The organism is a fungus, a yeast or an algae. Preferred yeasts include baker's yeast and methylotrophic yeast and preferred algae include red algae. Preferably the organism is an organism that belongs to a genus selected from *Saccharomyces, Pichia, Hansenula, Kluyveromyces, Candida, Brettanomyces, Debaryomyces, Tolrulopsis, Yarrowia, Galdieria, Cyanidium, Porphyridium, Cystoclonium, Audouinella, Cyanidioschyzon.*

An autolysate is herein understood to be a composition comprising autolytically solubilised cellular components such as amino acids, polypeptides, nucleotides, proteins, glycogen, sugars, B-vitamins, organic acids lipids and other components. Autolysis of organisms generally comprises an incubation of the organism's cells at an elevated temperature (30-50°C) for a prolonged period of time (3-18 hours) in the presence of a plasmolysing agent, such as e.g. NaCl, ethanol, ethyl acetate, chloroform or dextrose. During the incubation cellular components are hydrolysed by the cells endogenous hydrolytic enzymes, the cell wall breaks and disintegrates and releases the proteinaceous content into the aqueous environment. Insoluble cellular debris is removed by centrifugation and/or filtration and an autolysate comprising the above-mention soluble components is obtained.

The method preferably further comprisese the steps of: (a) growing an organism on a mineral medium which supports growth of the organism, whereby in the medium substantially all of the assimilable atoms, for at least one of H, C or N, are isotopically labelled, to produce labelled biomass; (b) extracting biomass of an organism with an organic solvent to produce an extract comprising lipids, whereby the organism is grown as in (a) or is grown as in (a) on a medium without isotopic substitution; (c) hydrolysing biomass of an organism grown as in (a) at a non-alkaline pH to produce a hydrolysate comprising amino acids; and, (d) composing the nutrient medium by combining the autolysate as obtained above with, the lipids as obtained in (b) and/or with the amino acids as obtained in (c) and adding further components necessary for growth of the mammalian or insect cells. the nutrient medium is a medium that is suitable for producing a protein by the mammalian or insect cells in culture. Preferably, further components necessary for producing proteins in mammalian or insect cells are added to the nutrient medium. Preferably substantially all of the assimilable atoms in the further components, for at least one ofH, C or N, are isotopically labelled. The non-alkaline pH, preferably is a pH below or equal to about pH 8.0.

As used herein, the term that a molecule is "substantially labelled" or that "substantially all" of the atoms of a particular element in a molecule or in a composition are "isotopically labelled" or "of a given isotopic form" means that the molecule or the composition comprising the molecule is sufficiently enriched with the desired isotope such that meaningful NMR spectral information can be obtained. In the case of NMR-active isotopes, such as ¹³C and ¹⁵N, the degree of enrichment will be such that three-dimensional structural information can be deduced from the NMR spectra. In general, in the context of the present invention, this means that 95% or more of the atoms of a given element will be in the desired isotopic form, preferably more than about 98, 99, 99.5 or 99.9%. In the case of enrichment with ²H alone, the degree of enrichment will be such that the labelled molecule does not produce an NMR signal sufficient to interfere with an analysis of an NMR-active species complexed to it or present in the sample to be analysed with NMR. In this case, the level of enrichment preferably is greater than about 70%, more preferably greater than about 80, 90, 95 or 98%. Alternatively, the level of ²H enrichment is such that the signals from the NMR-active nuclei, ¹H, ¹³C and ¹⁵N are enhanced or better resolved. In general this level of enrichment will range from about 20% to about 40, 50, 60, 70, 80 , 90 or 100%. It will be appreciated by the skilled person that although the terms "isotopically labelled" or "of a given isotopic form" in the context of the present invention generally refer to isotopes that are useful in obtaining NMR spectra. However, the invention is not necessarily limited thereto and may also pertain to other isotopes such as e.g. non-stable radioactive isotopes.

In the present invention, molecules are usually uniformly labelled, which means that 95% or more of the atoms of a particular element in a molecule are isotopically labelled to a specified degree or of a given isotopic form. Alternatively, it is disclosed, that molecules may be specifically labelled, which means that only substantially all atoms of a particular element in one or more specific positions in the molecule are isotopically labelled or of a given isotopic form. An example of specifically labelled amino acids and proteins consisting thereof is provided in WO99/11589, which discloses chemically synthesised amino acids that are used to produce proteins that are isotopically labelled in the backbone structure, but not in the amino acid side chains.

A biomolecule that is synthesised by the mammalian or insect cells may be any molecule that is synthesised by such cells, including e.g. proteins, polypeptides, peptides, nucleic acids such as poly-deoxynucleotides and poly-ribonucleotides, carbohydrates, lipids, metabolites and combinations thereof The biomolecule may be a molecule that is naturally synthesised by the mammalian or insect cells, or it may be a molecule that is synthesised by the cells as a result of genetic engineering. In the latter case the molecule will usually be a polypeptide or nucleic acid, however, metabolites that are produced as a result of genetic engineering, e.g. by introducing one or more genes coding for a particular enzymatic activities, are also included.

### Production of isotopically labelled biomass

The organisms that are grown in step (a) of the method of the invention are preferably grown on a mineral or chemically defined medium. Depending on the element to be isotopically labelled (C, N and/or H) the medium preferably contains a sole carbon and/or nitrogen source that is uniformly labelled with the isotope in question, more preferably 95% or more of the atoms in the sole carbon and/or nitrogen source are isotopically labelled. A variety of carbon and/or nitrogen containing compounds may be used for this purpose, including e.g. glucose and other sugars, small organic acids, urea and the like. Preferably however, carbon and/or nitrogen sources are used that contain only a single carbon or nitrogen atom, including the inorganic substrates CO₂, N₂, NH₃, NO₃⁻, NO₂⁻, HCO₃-, or organic C₁-compounds such as methanol, methane, methylamine, formaldehyde, and formic acid.

As a consequence, the organisms grown in step (a) preferably is a photosynthetic, chemolithotrophic or methylotrophic organism. The organism will therefore preferably be a microorganism although plants are not excluded. Plants may be grown as plants cells in *in vitro* culture or as such, in which case they may be grown in a confined space containing labelled carbon dioxide and on a substrate provide with mineral medium. The organisms are preferably grown autotrophically. When grown in culture the organisms are preferably grown in submerged culture preferably in a stirred fermenter with control of pH, temperature, light and supply of oxygen, carbon dioxide, carbon/nitrogen source and other nutrients as may be applicable and as is generally known in the art.

Suitable organisms to be grown in step (a) of the method include cyanobacteria (blue-green algae) and other photosynthetic bacteria, including non-oxygenic purple sulphur and non-sulphur bacteria, nitrogen-fixing bacteria, eukaryotic algae including green, red, as well as the brown algae, diatoms and other chrysophytes, as well as dinoflagellates, cryptomonads, and euglenoids, methylotrophic bacteria and fungi, including yeasts. Preferred organisms include *Rhodophyta, Cyanidiophyceae,* (in most cases it is mentioned as belonging to Rhodophyta), *Chlorophyta, Cyanophyta, Diatoms. Phaeophyceae,* and *Dinoflagelate* as sources of lipids, including polyunsaturated fatty acids (PUFA), lipid-soluble vitamins and sterols; methylotrophic bacteria as source of proteins (for further amino acids production); methylotrophic yeasts as source of proteins, (for further amino acids production), and vitamins; *Cyanophyta* as source of carbohydrates such as e.g. glucose, sucrose, fructose and vitamins; *Chlorophyta, Dinoflagelate* as source of glycerol and organic acids released in the medium; *Chlorobiaceae* (green sulphur) and *Rhodospirillaceae* (purple non-sulphur) bacteria as source of organic acids excreted in darkness into the medium.

The following examples are given of suitable genera within these classes of organisms are preferred for use in the method of the invention.

Cyanophyta (blue-green algae: Cyanobacteria): *Spirulina, Synechoccus, Anabaena,* or *Microcystis.* Chlorophyta (green algae): *Chlorella. Neochloris, Scenedesmus, Dunaliella, Haematococcus, Staurastrum,.* Rhodophyta (red algae): *Porphyridium, Cyanidium Cystoclonium,* or *Audouinella.* Cyanidiophyceae (thermophilie Rhodophyceae): *Cyanidium, Galdiera, Cyanidioschyzon.* Phaeophyta (brown algae): *Ectocarpus* or *Streblonema.* Algae from the group of heterokontophyta (heterokont chromophytes), prymneciophyta (haptophyta), bacillariophyceae (diatoms), chryptophyta, dinophyta (pyrrhophyta, dinoflagelletes)), euglenophyta (euglenoids), ciliates, and obligate hetrothrophic flagellates. Heterokontophyta: *Rhinomonas, Syncripta,* or *Heteroccus.* Prymnesiophyta (haptophyta): *Pseudoisochrysis, Phaeocystis, Prymnesium,* or *Emiliania.* Chrysophyta group including Bacilaariophyta class Bacillariophyceae (diatoms): *Phaeodactylum, Navicula, Nitzchia, Amphora, Centronella, Eucampia, Fragilaria,* Chrysophyta class Chrysophyceae (golden algae), Haptophyta class Haptophyceae. Chryptophyta: *Cryptomonas, Chroomonas, or Rhodomonas.* Dinophyta: *Peridinium, Oxyrrhis,* or *Crypthecodinium.* Euglenophyta: *Euglena* or *Astasia.* Ciliates: *Metopus, Cyclidium.* The phototrophic bacteria (Rhodospirilleae) are divided into two suborders, the purple bacteria (Rhodospirillineae) and the green bacteria (Chlorobiineae). Green sulphur bacteria (Chlorobiaceae): *Chlorobium* or *Pelodictyon.* Green non-sulphur bacteria (Chloroflexaceae): *Chloroflexus.* Purple sulphur bacteria (Chromatiaceae): *Chromatium, Amoebobacter,* or *Thiodictyon.* Purple non-sulphur bacteria (Rhodospirillaceae): *Rhodobacter, Rhodopseudomonas, Rhodophila, Rhodospirillum,* or *Rhodomicrobium.* Heliobacteria (Heliobacteriaceae): *Heliobacterium* or *Heliobacillus.* Aerobic photosynthetic bacteria: *Erythrobacter.* Methylotrophic microorganisms include obligate methylotrophic bacteria: *Methylobacillus, Methylophilus, Methylomonas, Methylococcus,* or *Methylobacter,* facultative methylotrophic bacteria: *Brevibacterium;* and yeasts in general, e.g. *Saccharomyces, Kluyveromyces* (e.g. grown on labelled glucose or ethanol) and preferably methylotrophic yeast: *Pichia* or *Hansenula.* Chemolithotrophic bacteria: *Ralstonia* or *Nocardia.*

Methods for producing labelled biomass are well known in the art as is evident from publications concerning growth of bacteria in the presence of labelled carbohydrate and salts (Kay, et al., supra), growth of bacteria in algal lysates (Chubb, R T., et al., Biochemistry, 30, 7718 (1991)), growth of yeast in algal lysates (Powers, R., et al., Biochemistry, 31, 4334 (1992)), growth of bacteria and yeast in labelled methanol (See, Moat, A. G. and Foster, J. W., Microbial Physiology, 2d Ed., John Wiley & Sons, New York (1988). p. 218) and the phototropic culture of algae in the presence of isotopically labelled CO₂ and/or N salts (Cox, J., et al., Stable Isotopes in Pediatric Nutritional and Metabolic Research, Chapman, T. E. et al., Eds., Intercept Ltd., Andover House, England (1990), p. 1615).

### Extraction of lipids

In step (b) of the method of the invention biomass as obtained in (a) is extracted with an organic solvent to produce an extract comprising lipids. The lipid extract may comprise (tri-, di- and/or mono-) glycerides, sterols, phospholipids, shingolipids, lipid-soluble vitamins and/or free fatty acids. Lipids, fatty acids etc are herein defined with reference to Christie (2003, In: "Lipid Analysis" 3-d Ed., The Oily Press) as fatty acids and their derivatives, and substances that are related biosynthetically or functionally to these compounds. In the context of the present invention the term lipids is defined as a group of naturally occurring compounds which have in common a ready solubility in organic solvents such as chloroform, benzene, ethers, and alcohols, and include e.g. such diverse compounds as fatty acids and their derivatives, steroids, carotenoids, terpens, bile acids and lipid soluble vitamins.

Prior to extraction the biomass may be recovered from the culture medium by means known in the art, including e.g. centrifugation or some form of filtration. Optionally, the biomass may then be washed using e.g. water or buffered and/or aqueous solutions of a given osmotic strength. Prior to extraction, the biomass may be dried using e.g. spray drying or freeze-drying (lyophilisation). The dried biomass may conveniently be stored prior to extraction. The spend culture medium from which the biomass has been removed may contain useful labelled compounds such as e.g. amino acids, secreted proteins, and carbohydrates. The spent culture medium is therefore preferably also dried by e.g. spray drying or lyophilisation. The dried residue may then be extracted and/or hydrolysed as described below for biomass. Alternatively the biomass and culture medium are not separated. In such instances the biomass and culture medium are preferable lyophilised together for concentration and/or storage and subsequent extraction and/or hydrolysis as described below.

In a preferred extraction method, the cells in the biomass will be comminuted by any suitable technique known in the art. These techniques e.g. include sonication but more preferably mechanical action is used such as crushing or grinding the biomass. Alternatively, the cells in biomass may also be treated so as to weaken their cell walls. e.g. by enzyme treatment. Comminution may be applied prior to extraction but more preferably it is applied during extraction in the solvent medium. This will increase the efficiency of extraction. Various types of known extraction or rather comminution apparatuses can be used for the purpose of disintegrating the cells in the biomass, including the wet-process pulverising machines, such as ball mills, frictional disk mills, Henshel mixers, French presses and the like. Preferably, the cells in the biomass are at least partly destroyed or broken by compressive or frictional mechanical force in the comminution apparatuses. It will be noted, however, that the cells should not be disintegrated to an excessive degree that will result in particles too fine to be easily separated from the solvent mixture.

The basis of any extraction method is to bring the biomass into contact with a suitable extraction medium for a period of time, and then to separate the mixture of the biomass and extraction medium into (1) the undissolved components of the mixture (i.e. the extracted biomass) and (2) the dissolved extract (i.e. the extraction medium with dissolved components extracted from the biomass). Under ideal extraction conditions solid substances should dissolve in a solvent rapidly and totally in a short period of time. However, this cannot be assumed when dealing with complex mixtures of substances, let alone substances in algal cells bound to membranes and associated with protein complexes. For more efficient extraction the biomass and the extraction medium are mixed by some form of shaking, stirring, mixing or homogenisation, including the mechanical means indicated above. Separation of the mixture may be performed by various means commonly known in the art, including sedimentation, centrifugation and filtration. The separated extracted biomass may then be extracted again with the same or a different extraction medium. Various dissolved extracts may be combined and the extracted components may be recovered from the dissolved extract by various means known in the art, including e.g. distillation or (flash) evaporation of the solvent(s) of the extraction medium.

Suitable solvents for the extraction of lipids from the biomass are lower aliphatic alcohols or lower aliphatic hydrocarbons whereby lower aliphatic means C₁ - C₈. However, in principle any other organic solvent, including e.g. acetone, halogenated lower aliphatic alcohols or hydrocarbons, as well as lower aliphatic ethers may equally be applied. Likewise mixtures of organic solvents may be applied. Suitable methods will generally include the use of the appropriate solvent(s) under conditions which will prevent oxidation of the unsaturated lipids, e.g. performing the extraction under vacuum or under an inert gas such as nitrogen, argon or the like. Alcohols are preferred as solvents for the extraction over acetone. However, since alcohols are known to promote the formation of allomers of chlorophyll and since chlorophylls have commercial value, chlorophyll-containing biomass (such as algal biomass) is preferably extracted with acetone as solvent.

Although not critical, the amount of solvent used in the present invention should be in the range of about 2 to 7 parts by weight, preferably about 3 to 6 parts by weight per part of biomass on a dry basis. It is preferred that water be present with the solvent during extraction. The amount of water to be used with the solvent should be in the range of about 0.2 to 0.7 parts by weight or, preferably, from 0.3 to 0.6 parts by weight per part by weight of the solvent on a anhydrous basis. Such an amount of water can be supplied separately in a calculated weight when the biomass is completely dry, but the amount of separate addition of water should be reduced when the biomass contains water as is the case when a wet cake of biomass bodies is used as obtained by centrifugation or filtration for removal of the culture medium. In case an aqueous suspension of microorganisms is extracted, the ratio between the amount of the aqueous suspension used and the extraction solvent used is not in itself critical. Ratios by weight of between 1:1 and 1:100 are generally employed. Ratios between 1:2 and 1:50 are customarily used, and finally working ratios of between 1:5 and 1:20 are preferably employed.

Several methods for extracting lipids from (microbial) biomass are known in the art. One conventional technique e.g. involves homogenising the biomass in a solvent mixture of chloroform and methyl alcohol. Another suitable method for extracting lipids from biomass is described in U.S. Pat. No. 4,857,329. This patent describes the extraction of lipids from cells of a fungus of the genus *Mortierella* by first grinding the cells in the presence, or absence, of a hot alcoholic solvent, and then extracting the alcohol-treated ground cells with a solvent, preferably in a supercritical state, or a mixture of a solvent in a supercritical state with a lower aliphatic alcohol, or a lower aliphatic hydrocarbon. The lower aliphatic alcohol is preferably one having a boiling point of about 40°C to about 120°C (e.g. ethanol, propanol, isopropanol, butanol, and isobutanol). Preferred lower aliphatic hydrocarbons include butane, pentane, hexane, heptane, and cyclohexane. Preferred process conditions include temperatures from about 35°C to 90°C and pressures from about 200 to 600 kg/cm². Another suitable method is disclosed in U.S. Pat. No. 4,870,011. The method comprises mechanical disintegration in two steps, first with an alcohol/water mixture to give a fraction rich in polar lipids, and then with a hydrocarbon solvent, e.g. hexane, to give a fraction lean in polar lipids. A particularly preferred solvent system is a mixture of hexane and isopropanol at a volume to volume ratio of about 3 to 2. Preferred extraction methods for extraction of lipids and fatty acids use mixtures of chloroform and methanol. Preferably chloroform is substituted by the less toxic dichloromethane, the latter also being easier to remove by evaporation.

Lipids may be sensitive to oxidation, light, heat, acids and alkali. During extraction lipids may thus undergo undesired structural changes due to e.g. oxidation or isomerisation. The skilled person will know how to take the necessary precaution to reduce such undesired changes during the extraction process.

Lipids are generally water-insoluble organic substances found in cells, which may be extracted by nonpolar solvents. There are several major classes and subclasses of lipids, most of which may occur in different molecular species, dependent on the structure of their fatty acid components and each have preferred extraction methods. Acylglycerols (also referred to as neutral fats or glycerides) are preferably extracted with ether, chloroform, benzene or ethanol; phosphoglycerides (e.g. PEA=cephalin, PC=lecitin) are preferably extracted with mixtures of chloroform/methanol; steroids, in particular sterols are preferably extracted with chloroform, ether, benzene, or hot alcohol; terpens (minor lipid components of cells) which include carotenoids, are preferably extracted using water-miscible solvents such as acetone, methanol or ethanol; fat-soluble vitamins extractable may generally be extracted with organic solvents; sphingolipids (cerebroside), glycolipids (glycosyldiacylglycerols, cerebroside, ganglioside), prostaglandins and cholesterol may extracted as described by Christie (*supra*).

Pigments, such as chlorophylls and carotenoids are preferably extracted using water-miscible organic solvents (see above).

The organisms for production of a lipid extract are preferably selected on the basis of high lipid contents and/or the specific lipid composition thereof. Nagashima reviews the major fatty acid distribution in various algae classes (1994, In: "Evolutionary Pathways and Enigmatic algae: Cyanidium caldarium (Rhodophyta) and related Cells ", J. Seckbach, ed., Kluwer academic Publishers, p. 201-214). Most algae can serve as source of C₁₆ and C₁₈ series of fatty acids, as well as, C₁₃ unsaturated fatty acids. *Rhodophyta, Chryptophyta, Chromophyta* and *Haptophyta* may serve as source of C_{20:4} and C_{20:5}.

The distribution pattern of unsaturated fatty acids varies within the individual algae classes. *Chlorophyceae* is a preferred source for types C_{16:3 (n-3)}, C _{16:4 (n-3)}, *Rhodophyceae* is a preferred source for types C_{20:4}(_{n-6).} C_{20:5 (n-3)}, and *Phaeophyceae* is a preferred source for types C_{18:3(n-3)}, C_{18:4(n-3)}, C_{20:4(n-6)}, C_{20:5(n-3)} (Takagi, et al., (1985) Yukagaku, 34, 1008-1012., see also Banaigs et al. (1984, Phytochemistry 23: 2951-2952).

Red algae in general (e.g. *Cystoclonium purpureum*) are preferred sources for long chained, polyunsaturated fatty acids (i.e. C₁₈ and longer) and their derivatives (Pohl and Zurheide, (1979) In Hoppe, H.A., Levring, T., Tanaka, Y. Eds Marine Algae in Pharmaceutical Science. Walter de Gruyter, Berlin, pp. 437-523). *Porphyridium* species such as e.g. *P. aerrugenium* (fresh water) and *P. ruentum* (salt-water) are preferred sources for arachidonic acid C_{20:4(n-6)} (36% of the total fatty acids) (Arad, 1986, Int. Indus. Biotechnol. 7: 281-283; Arad et al., 1985, In: "Biosalinity in Action: Bioproduction with saline water", Pasternak, D., San Pietro A eds, Nijhoff Publishers, pages 117-128; Gudin, 2002, In: "Algal Biotechnology. A sea of opportunities", Book of Abstracts. Universidad de Almeria, Servicio de Publicaciones).

Dinophyta, such as e.g. *Crypthecodinium cohnii* are preferred sources for long-chain polyunsaturated fatty acids (PUFA) such as DHA- C_{22:6(n-3)} (see e.g. Borowitzka, 1999, J. Biotechnology 70: 313; U.S. 5,711,983; U.S. 4,670,285; WO91/14427; Kyle et al., 1992, In: "Biotechnology and nutrition", Bills, D.D., Kung, S.D. Eds., Butterworth-Heinemann, Boston, pages 451-468; Gudin, Sijtsma et al. and Mendoza, 2002, In: "Algal Biotechnology, A sea of opportunities", Book of Abstracts. Universidad de Almeria, Servicio de Publicaciones).

Heterotrophic diatoms such as *Nitzschia, Cyclotella, Navicula* are preferred source for EPA - C_{20:5(n-3)} (see WO 91/14427). Another source for the EPA is marine diatom *Phaeodactylum* (see Belarbi, E.H. et al., Acien, F.G. et al., Sanchez, M. et al., and Cini Zitteli, G., et al. in Algal Biotechnology. A sea of opportunities. Book of Abstracts. Universidad de Almeria, Servicio de Publicaciones) and marin diatom *Tetraselmis* (Ceron, M.C.. et al 2002, in the same book).

Chlorophyta such as *Chlorella. Dunaliella, Haematococcus, Parietoch*/*oris* are source for PUPA (see Cohen, et al., Gudin. 2002, In: "Algal Biotechnology. A. sea of opportunities". Book of Abstracts. Universidad de Almeria, Servicio de Publicaciones). Cyanophyta *Spirullina* may used as a source for PUFA's.

Green algae *Dunaliella* is a source of glycerol (20-40% of the algal organic weight) and intracellular glycerol concentration is directly proportional to the extracellular salt concentration and is dependent on the conditions of cultivation. Below 25 °C little or no glycerol was found in the medium. Above 25° C, the rate of glycerol release into the medium gradually increased and above 40° C it increases dramatically, so that by 50 °C the alga loses its glycerol into the medium within a few minutes (Wegmann, *et al.,* 1980).

Thus, blue-green algae are a preferred source for C₁₆ fatty acids, green algae for C₁₈ fatty acids, and red algae and diatoms for C₁₆-C₂₀ fatty acids. Higher algae in general, i.e. eukaryotic algae, are preferred sources for phosphatidyl-choline (lecithin).

The skilled person is aware that by varying growth conditions, the actual composition of lipids produced by a given organism may be influenced. It is e.g. well documented that an increase in temperature causes a corresponding decrease in the amount of unsaturated fatty acids. A further example of the influence of temperature on the lipid composition is *Cyanidium caldarium* (see Kleinschmidt and McMahon, 1970, Plant Physiol. 46: 290-293). *C. caldarium* cells grown at 20°C contained significantly larger quantities of glycolipids (mono- and di-galactosyldiglyceride) and phospholipids (phosphatidyl-choline and phosphatidyl-ethanolamine) as compared with cells grown at 55°C. Similarly, cells grown at 20°C contained polar lipid components in which the degree of unsaturation is three times higher than in cells grown at 55°C. *C. caldarium* is preferably cultured at 25° C.

In general, for production and isolation of natural products from algae, the reader is referred to Cresswell, Rees and Shah (1989, Algal and Cyanobacterial Biotechnology. Longmann & Scientific technical), Cohen (1999, Chemicals from microalgae. Taylor & Francis) and the Book of abstracts "Algal biotechnology. A sea of opportunities" (2002, supra).

Algae generally produce wide spectrum of sterols. The distribution of sterol in algae is e.g. reviewed by Patterson (1991, In: "Physiology and Biochemistry of Sterols", Eds. Patterson, G.W. and Nes, W.D., American Oil Chemists Society, Champaign. Ilinois, p.p. 351-354). Ergosterol and sitosterol may e.g. be obtained from most algal divisions as well as from higher plants. Red algae, such as e.g. *Rhodophyta,* are preferred sources for cholesterol, and may, in addition serve as source of C₂₇, C₂₈, C₂₉ sterols (with the C₂₈ as predominant) and also as source of sitosterol, methylene-24 cholesterol and campesterol (24-methylcholesterol). The *Chlorophyta* (green algae) are preferred sources of sitosterol, (*Chlorella*), campesterol, poriferasterol (24-ethylcholesta-5,22-dienol), ergosterol (24-methylcholesta-5,7,22-trienol) and other sterols. Cyanobacteria (blue-green algae) are preferred sources for a large spectrum of sterols including cholesterol, ergosterol, chondrillasterol, sitosterol and campersterol and other 24-alkylsterols. Brown algae are preferred sources of fucosterol (24(E)-ethylidenecholesterol). Cyanidiophyceae (Cyanidium) are preferred sources of ergosterol, β-sitosterol, and campesterol. Thus algae could be considered as a source to obtain uniformly labelled sterol from lipid extracts. Standard procedures for their isolation are generally known in the art. Two methods have frequently been used for the isolation of sterols from plant or algal extracts. A substantial amount of sterol can be precipitated from petrol solutions that are left overnight at -10° C, but more efficient procedure involves precipitation of the sterols as their digitonides (see also Volkman, J.K Sterols in microorganisms. Appl. MicrobioL Biotechnol (2003) 60: 495-506, Patterson, G.W. (1991) In: Physiollogy and Biochemistry of Sterols (Eds, Patterson, G.W. and Nes, W.D.) American Oil Chemists Society, Champaign, Ilinois, pp. 118-157).

### Amino acid hydrolysates

In step (c) of the method of the invention biomass of an organism grown as in step (a) is hydrolysed to produce a hydrolysate comprising amino acids. The biomass is preferably hydrolyses at a non-alkaline pH, preferably a pH below 8.0. The proteinaceous material in the biomass may be hydrolysed enzymatically, by acid treatment or by a combination of both. Many procedures for the hydrolysis of proteins have been published, including hydrolysis with hydrochloric acid, methanesulphonic acid and enzymatic hydrolysis.

In case of enzymatic hydrolysis, preferably the proteolytic enzymes are immobilised on a solid carrier so that they may be conveniently removed from the hydrolysate, preferably with other insoluble components, e.g. by sedimentation, centrifugation or filtration. Alternatively, it may be convenient to acidify the hydrolysate subsequent to the enzymatic reaction. This has the advantage of denaturing and precipitating the enzyme, which can then be removed from the hydrolysate, e.g. by centrifugation or filtration. Methods for enzymatic hydrolysis of proteins or proteinaceous material in biomass are described by Milligan and Holt (1977, Adv. Exp. Med. Biol. 86B: 277-284) and by Bergmeyer (1984, In: "Methods of enzymatic analysis: Enzymes 3: peptidases, proteinases, and their inhibitors", VCH Publishing, 3-d ed.). Commercially available mixtures of endo- and exo-protease that can be used in the present invention for enzymatic hydrolysis are, e.g. Sumizyme^{™}. FP, Sumizyme^{™} LP-proteases (both from Shin Nihon, Japan), Flavourzyme^{™} protease (Novo Nordisk A/S, Denmark) and Protease M^{™} Amano (Amano, Japan). Other comparable enzymes having similar properties can be used as well. In view of the acidic pH optima of fungal enzymes, the endo- and exo-protease mixtures preferably are obtained from an *Aspergillus* species, especially a species such as *A.oryzae* or *A.sojae,* although enzymes from other *Aspergillus* species, or indeed, other fungal species, similarly can be employed. Mixtures of these proteases, e.g., with other proteases such as for example, Pescalase^{™} (DSM, The Netherlands) protease, which is a bacterial endoprotease, may also be used. A preferred enzyme for enzymatic hydrolysis of proteins or proteinaceous material in biomass is Pronase as obtainable from Fluka (Buchs SG, Switzerland).

In the present method, acid hydrolysis is however preferred. Methods for acid hydrolysis of biomass for obtaining labelled amino acids are extensively described in WO 94/18339. Basically these methods include the use of a strong mineral acid, such as hydrochloric acid, nitric acid or sulphuric acid. More preferred are however sulphonic acids such as p-toluenesulphonic acid or methanesulphonic acid, the latter being most preferred. The acid concentration may vary, depending upon the nature of the protein substrate, but in general is sufficient to effect complete hydrolysis. Typically, acid concentrations range from about 1N to about 8N, preferably from about 4N to about 7N, more preferably from about 5N to about 6N. The acid hydrolysis is preferably carried out under non-oxidising conditions. These conditions may be achieved by conducting the reaction in vacuo or by purging with an inert gas such as nitrogen, argon or the like. The protein to be hydrolysed may be added to the hydrolysis medium at a concentration of between about 50 g/l and 500 g/l, preferably at a concentration of between about 100 g/l and 250 g/l. The hydrolysis is carried out at a temperature and for a time sufficient to effect substantially complete hydrolysis, while at the same time minimising racemisation or the loss of labile amino acids. The temperature of the hydrolysis generally ranges from about 90 to 140°C., but in order to minimise the racemisation of amino acids the temperature is preferably in the range 100 to 130°C., more preferably between 110 and 120°C, with 115°C particularly preferred. The time of hydrolysis may be in the range of 10 to 72 hours, depending on the protein to be hydrolysed. Preferably a hydrolysis time of about 22 hours is used. During the hydrolysis reaction, amino acids that are susceptible to oxidation are preferably protected by the presence of a reducing agent. Preferably, a strong sulphhydrylcontaining reducing agent is employed, such as thioglycollic acid (Fasman, G. D., Ed., Practical Handbook of Biochemistry and Molecular Biology, CRC, New York (1989), p. 106). The purpose of the reducing agent is not just to protect the vulnerable tryptophan and histidine residues. If thioglycollic acid is used, it can easily be subsequently removed according to the procedure of the invention. The reducing agent is employed at a concentration in the hydrolysis mixture sufficient to prevent substantial destruction of tryptophan and histidine. For thioglycollic acid, such concentration generally ranges from about 1 to about 7% v/v, preferably from about 3 to about 15% v/v. More preferably or additionally destruction of tryptophane and histidine may be reduced or prevented by the inclusion of "suicide bases" like tryptamine and imidazole (see e.g. Hansen et al., 1992, Biochem. 31: 12713). Each tryptamine and imidazole are preferably added at 10 - 15 g per litre acid hydrolysis mixture. Preferred hydrolysis conditions e.g. include the hydrolysis of 10 g of biomass, preferably free of lipids and pigments (see below), in 150 ml of 4 M methanosulphonic acid at 115°C for 22 hours under vacuum and in the presence of 2 g tryptamine and 2 g imidazole.

Since glutamine and asparagine may be significantly deaminated under the above hydrolysis conditions, they are preferably added from different sources including e.g. chemical or enzymatic synthesis, enzymatic hydrolysis of isotopically labelled biomass or proteins that are rich in glutamic acid and/or aspartic acid, or they may be obtain directly from fermentation of overproducing strains (see e.g R Faurie., J. Thommel. 2003 Microbial production of L- amino acids. Springer Verlag).

In a preferred embodiment, the proteinaceous material in the biomass is first subjected to enzymatic hydrolysis to partially hydrolyse the proteinaceous material. After removal of the proteolytic enzymes the material is further hydrolysed to completion with acid as described above. When enzymatic and acid hydrolysis are combined, preferably a mixture of proteolytic enzymes is used which at least comprises endoproteases, which are readily available as relatively pure and cost effective products. Preferably aspecific endoproteases are used. Suitable commercially available composition comprising endoproteases are mentioned above.

Preferably in the method, the biomass is extracted with an organic solvent to remove lipids and pigments from the biomass, prior to its hydrolysis. The advantage of extracting lipids and particularly pigments from the biomass prior to hydrolysis is that these compounds may be toxic to insect or mammalian as such or may be converted into toxic compounds during hydrolysis, or that they may interfere with hydrolysis. Lipids and pigments may be extracted from the biomass as described hereinabove. Preferably the extraction is optimised for at least efficient extraction of pigments. The biomass is preferably extracted more than once whereby separate extraction steps may be optimised for the extraction lipids and for the extraction of pigments. Preferred conditions for extraction of pigments are described above.

Preferably insolubles are removed from the hydrolysate by centrifugation or filtration. Amino acids may be isolated from the hydrolysate e.g. by reverse phase- or ion exchange-chromatography as inter alia described in Egorova et al. (1995, J. Chrom. Biomed. Appl. 665: 53-62) and WO 94/18339.

Finally, in the method of the invention, a nutrient medium is composed by combining lipids as obtained in (b) with amino acids as obtained in (c) and adding further components necessary for growth of the mammalian or insect cells. The nutrient medium may be composed of lipids and amino acids obtained from one and the same organism or from at least two different organisms. Preferably, each the amino acid hydrolysate and the lipid extract are obtained from at least two different organisms. Thus, one or more organisms may be selected for production of an amino acid hydrolysate and one or more different organisms may be selected for the production of a lipid extract.

The organisms for production of an amino acid hydrolysate are preferably selected on the basis of high protein contents and/or amino acid composition thereof. Suitable organisms for this purpose are mentioned above and include e.g. green algae like *Spirulina* with a protein content of 70% dry weight, or methylotrophic bacterial such *as Methylobacillus.* Russian Patent SU 989867 discloses a bacterial strain ot the species *Methylobacillus methylophilus* (YSB-932) with a protein content of 80% dry weight.

Further preferred organisms for production of an amino acid hydrolysate include yeasts in general, e.g. *Saccharomyces*, *Kluyveromyces*, *Catidida*, *Hansenula*, *Pichia*, *Brettanomyces*, *Debaryomyces*, and *Tolrulopsis*. The preferred genera include *Pichia*, *Hansenula*, *Saccharomyces*. Particularly preferred are methylotrophic yeast such as *Pichia pastoris* and *Hansenula polymorpha,* which can grow on a broad range of carbon sources, including glucose, sucrose, trehalose, maltose, glycerol, erythritol, xylitol, mammitol, methanol and ethanol. A preferred method for producing an amino acid hydrolysate from yeast may include autohydrolysis of yeast biomass as e.g. described in US 4,165,391 and in Lukondeh et al. (2003, J. Ind. Microbiol. Biotechnol, 30: 52). Stable isotope labelling of yeast and the preparation of extracts therefrom is disclosed in JP 6261743 (1994).

A yeast autolysate is a concentrated product, which contains autolytically solubilised cellular components such as amino acids, polypeptides, nucleotides, proteins, glycogen, sugars, B-vitamins, organic acids and other components. Autolysis of yeasts generally comprises an incubation of yeast cells at an elevated temperature (30-50°C) for a prolonged period of time (3-18 hours) in the presence of a plasmolysing agent, such as e.g. NaCl, ethanol, ethyl acetate, chloroform or dextrose. NaCl is used at a concentration of 5-20% w/v, preferably from 2 to 10 % w/v and the solvents are used at a concentration of from 1 to 10 % v/v. During the incubation cellular components are hydrolysed by the yeast's endogenous hydrolytic enzymes, the cell wall breaks and disintegrates and releases the proteinaceous content into the aqueous environment. Insoluble cellular debris is removed by centrifugation and/or filtration and an autolysate comprising the above-mention soluble components is obtained.

In the present invention, preferably a yeast species as indicated above is cultured in a nutrient medium containing a carbon source and a nitrogen source (¹³C - and/or ¹⁵N stable isotope labelled), inorganic salts, etc., and cells are harvested by centrifugation and washed with water appropriately, and the resulting live yeast cells are used for producing extract. The water component can be pure water, water containing dilute salts or the natural liquor of the fermenter/culture. The yeast cells are suspended in water at the concentration of preferably about 5% to 15% on dry weight basis and subjected to a heat shock 50-75 °C, preferably 65-70°C, for about 30 seconds minute to induce cracks in the cell walls and to induce autolysis. In order to maintain the activity of proteases and other hydrolytic enzymes involved in autolysis, treatment for a longer time and a temperature above 65 °C is not preferred and rapid cooling after the heat shock is preferred. Subsequently the yeast cells are allowed to autolyse at a pH of 6.5-10.0, preferably a pH of 7.5-8.0 and at 10% w/v NaCl. The pH may be adjusted before or after the heat shock. To adjust the pH a dilute ¹⁵N labelled ammonium hydroxide or sodium hydroxide, potassium hydroxide can be employed. The suspension of heat shocked yeast cells in water is then kept at about 30-50°C, preferably 40-45°C for about 3-12 hours to allow for enzymatic autolysis. The soluble components are then recovered by centrifugation or filtration to produce a yeast autolysate that may be lyophilised for long term storage.

In the context of the present invention, the terms "yeast extract", "yeastolate", yeast hydrolysate and/or "hydrolysate comprising amino acids" (produced from yeast) are understood to include such amino acid-containing hydrolysate produced by hydrolysis methods which may involve each chemical hydrolysis, enzymatic hydrolysis by both exogenous and endogenous enzymes, the latter also referred to as autolysis.

### Further nutrients

Isotopically labelled glucose may be isolated from cyanobacteria or may obtained commercially. Salt stress conditions (e.g. growth in 0.5 M NaCl) may be applied to increase the carbohydrate content of biomass of e.g. *Spirulina.*

Organic acids may suitably be obtained from Dinoflagelates, which produce up to 69% organic acids. Dinoflagellates (genus Symbiodinium) are unicellular motile algae with the peculiar feature of permanently condensed chromosomes. Dinoflagellates are source of glycerol and organic acids. In most symbiotic associations the dinoflagellates are located intracellularly in the gastrodermal layer and are surrounded by a membrane of host origin. The major compounds released are glycerol (21-95%), organic acids, (0-69%), glucose (0.5-21 %) and alanine (1-9%), Differences between *Symbionidium* isolated from different hosts are exemplified by *Zoanilnus pacifica* and the *Seyphozoam Rhizostoma.* Symbionts from Zoanthus release 42% of their photosynthate, of which 95% is glycerol and 3% organic acids, whereas symbionts of *Rhizostoma* release 20% of their photosynthate of which 21 % is glycerol and 69% organic acids (Trench, 1971 Proc. R. Soc. Lond. B, 177, 251-264.

Green sulphur bacteria *Chlorobium,* when in incubated in dark, excretes organic acids such as acetate, propionate, caproate and succinate (see Sirevag, 1995, In: "Anoxigenic photosynthetic bacteria", Eds. Blankenship, R.E., et al, Kluwer Academic Publishers, p. 879.) Organic acids may also be applied as a carbon source for the

The nutrient medium is preferably composed of various amino acid hydrolysates, lipids extracts, carbohydrates and organic acids that may be obtained from microbial cultures as described above. The medium may further be supplemented as necessary with chemically synthesised or commercially available components. Various hydrolysates, lipid extracts and carbohydrate and organic acid preparation have been screened and tested in order to find the most significant effect of the medium components and their concentration on final cell concentration and/or protein production levels.

A preferred nutrient medium for isotopic labelling of biomolecules produced in insect or mammalian cells according to the invention comprises:
(a) a mixture of inorganic salts;
(b) a source of isotopically labelled amino acids;
(c) an isotopically labelled energy source, usually in the form of carbohydrates such as glucose
(d) a source of lipids
(e) a protective agent
(f) (optionally) vitamins and /or organic compounds required at low concentrations;
(g) (optionally) organic acids
(h) (optionally) trace elements.

Mixtures of inorganic salts for incorporation into cell media are well known in the art. The mixture of inorganic salts preferably provide for a physiological ionic strength and pH buffering capacity. A suitable mixture of inorganic salts for insect cells is e.g. the salt mixture as known from Grace's medium or Schneider's medium. A suitable mixture of inorganic salts for mammalian cells is e.g. the salt mixture as known from Dulbecco's Modified Eagle's medium (D-MEM) (Price, P.J., et al., 1995, Focus, 17: 75), BME (Basal Medium Eagle, see Eagle, H. (1965) Proc. Soc. Exp. Biol. Med. 89: 362), F-10, F12 Nutrient mixture (Ham, 1963, Exp. Cell. Res. 29: 515) and their modifications, or CHO-SSFM1 median (Gibco). Preferably, in case the mixture comprises inorganic salt comprising one of the elements H, C, or N. substantially all of the atoms in the salts are isotopically labelled in accordance with the type of isotopic labelling of the other components such as the amino acids. E.g., NaHCO₃ is present in BME at 2.2 g/l, and in D-MEM at 3.7 g/l. In addition D-MEM also contains Fe(NO₃)x 9H₂O at 0.05 mg/l or 0.1 mg/l. Most if not all insect cell culture media are based on the Grace's formulation (Grace, 1962, Nature 195: 788) and also contain NaHCO₃ at a concentration of 0.35-0.7 mg/l. IPL-41 contains (NH₄)₆ (Mo₇O₂₄x4H₂O) at 0.04 mg/l.

Similarly, the composition of the trace elements (defined as inorganic compounds or naturally occurring elements that are typically required at very low concentrations, usually in the micromolar range) for optional incorporation into the nutrient medium and their final concentrations in the medium are well known in the art for both insect and mammalian cells (see e.g. Thilly, 1986, In: "Mammalian cell technology", p. 109 , Butterworths Publishers; Vlak et al., 1996, In: "Insect cell cultures: fundamental and applied aspects" Volume 2, Kluwer Academic Publishers; and Perekh and Vinci, 2003, In: "Handbook of industrial cell culture: mammalian, microbial, and plant cells", Humana Press).

The source of energy for insect or mammalian cells in culture will usually comprise carbohydrates, preferably glucose. Isotopically labelled glucose or other sugars such as mono- or di-saccharides could be obtained commercially or isolated from the biomass of algae. A preferred of biomass as source of carbohydrate such as glucose is green algal biomass, preferably grown under salt stress conditions.

### The source of amino acids

The source of isotopically labelled amino acids for the nutrient medium of the invention is preferably selected from a wide variety of hydrolysates of biomass or protein products, which hydrolysates may be used alone or in combination. Preferred, hydrolysates are obtained from delipidised or solvent extracted algal, bacterial or fungal (yeast) biomass obtained as described above. These hydrolysates replace expensive isotopically labelled amino acids and (to some extend) vitamins. The source of amino acids may be supplemented with individual amino acids that are present in insufficient amounts in the hydrolysates obtained from other sources such as chemical or enzymatic synthesis or fermentation. Such amino acids in particular may be glutamine, arginine, cysteine, histidine and tryptophane. However, trace amounts of tryptophane (after acidic hydrolysis) may be sufficient for the growth of insect and mammalian cells (as shown by Hansen et al., *supra*). Preferably the source of amino acids comprises an isotopically labelled yeastolate, obtained from yeasts in general or more preferably from methylotrophic yeasts (such as *Pichia* or *Hansenula*), isotopically labelled algal hydrolysate (*Cyanidium*, *Spirulina*), and/or isotopically labelled hydrolysate obtained from methylotrophic bacterial biomass (*Methylobacillus*).

A serum-free medium for large-scale culture of insect (Spodoptera frugipedra) cells was reported in Maiorella et al., (1988, Biotechnology 6: 1406-1410). In addition to basal medium, the medium contained yeast extract, cod liver oil PUFA methyl esters, cholesterol and Tween. In the present invention cod liver oil can be replaced by algal and bacterial lipid extracts that are preferably isotopically labelled.

Patent WO 92/05247 discloses serum-free media for the culture of insect cells, which contains basal medium to which is added a yeast hydrolysate, and albumin or dextran. In accordance, in the present invention a yeast hydrolysate may be added in the amount from 1 to 10 g/l.

The concentration of the source of amino acids in the medium of the invention may be as high as the sum of the highest allowable concentrations of the individual hydrolysates whereby the highest allowable concentration of each hydrolysate is that concentration at which is the hydrolysate is non-toxic or inhibitory to cell growth. The highest allowable hydrolysate concentration may vary not only with the particular hydrolysate preparation used but also with the particular cell line that is grown in the medium. The skilled person can easily determine the highest allowable concentration, e.g. by adding increasing amount of a given hydrolysate preparation to a cell line growing in regular medium.

Typically, preferred final concentrations of the source of amino acids in the medium of the invention can range from about 1 to 15 gram of source of amino acids (dry weight) per litre of medium, more preferably the final concentration ranges from about 2 to 10 g/l.

A preferred source of amino acids for a nutrient medium of the invention for insect cells, such as e.g. *Spodoptera frugiperda* cell line Sf9, comprises 0 to 7 g/l isotopically labelled algal hydrolysates, preferably 2.5 - 4 g/l, and/or 0 to 7 g/l isotopically labelled bacterial hydrolysate, preferably 1.5 - 3 g/l, and/or 0 to 3 g/l isotopically labelled fungal/yeast hydrolysate, preferably 5-7 g/l.

A preferred source of amino acids for a nutrient medium of the invention for mammalian cells, such as e.g. CHO cells, comprises 0 to 10 g/l isotopically labelled algal hydrolysate, preferably 4 - 6 g/l, and/or 0 to 10 g/l isotopically labelled bacterial hydrolysate, preferably 4 - 6 g/l, and/or 0 to 12 g/l isotopically labelled fungal/yeast hydrolysate, preferably 7 - 9 g/l.

Preferably, the hydrolysates that make-up a source of amino acids for the nutrient medium of the invention are purified by ultrafiltration to remove residual proteases used during the production of the peptone product, endotoxins and other high molecular weight components that could interfere with the production and/or purification of (recombinant) proteins expressed by the host cells The peptone fractions are preferably prefiltered and then ultrafiltered through a membrane with a molecular weight cut-off selected to be smaller that the molecular weight of the recombinant or viral product to facilitate later purification, preferably a 2000-15000 molecular weight cut-off membrane, more preferably, through a 10,000 molecular weight cut-off membrane such as PM10 membrane (Amicon). The ultrafiltration process is preferably carried-out in a cross-flow filtration apparatus, for example, in a pressurised stirred cell for small scale or in a hollow fiber cartridge or plate and frame device for large scale. The ultrafiltrate may be filter sterilised prior to addition of the basal medium to which other components of the media of this invention are also added.

Stock solutions of isotopically labelled hydrolysate are preferably prepared in the concentration of 10-15% (dry weight/v) for algal hydrolysates, 15-20% for fungal/yeast hydrolysates and 15-18% for bacterial hydrolysates.

Several lots of each lysate are tested for their growth promoting properties for e.g. Sf9 or CHO cells.

It is to be understood that the hydrolysates, in particular autolysates, as used may still contain oligopeptides in addition to free amino acids. In some hydrolysates up to about 20% of the total amino acid content is present in the form of oligopeptides. The free amino acid concentrations in the culture medium can vary during the culturing of the cells and may e.g. depend on peptidases/proteases released by the cells. This has to be taken into account when amino acid consumption (expressed as percentage of the initial content in culture medium) is determined. The concentration of the source of amino acids to be applied in the medium will depend on such factors as particular fractions of sources of amino acids that are employed, the nature of the cell line to be cultured, the level at which a given peptone becomes toxic or inhibitory to cell growth. The optimal concentration for each combination of cell line and source of amino acids may be determined empirically.

Generally, the total amount of source of amino acids in the nutrient medium will range from about 8 to 30 g/l, more preferably from about 12 to 24 g/l.

The relative concentrations of each individual amino acid and vitamin can be adjusted according the needs of the particular cell line. For example, although glutamine is essential for some insect cell lines, it is known in the art that other cell lines can grow without glutamine and may be able to synthesise this amino acid from precursors (see e.g. Mitsuhashi, 1987, Appl. Entomol. Zool. 22: 533-536). Depending on the intended use of the media the concentrations of the individual free amino acids and vitamins can be adjusted to accommodate the known characteristics of the various insect or mammalian cell lines.

### The source of lipids

The lipid requirements of insect cells are generally similar to those of mammalian vertebrates, with the exception that the insect cells do require contain a source of cholesterol. The source of lipids for incorporation into the nutrient medium of the invention preferably comprises that are essential for the growth of the insect or mammalian cells. Preferably these lipids are isotopically labelled lipids but this is not essential for the invention. The source of lipids preferably at least comprises (1) fatty acids, (2) steroids, and (3) lipid soluble vitamins.

The fatty acids in the source of lipids may be present in various forms such e.g. triglycerides, free fatty acids or alkyl esters of fatty acids (preferably C₁-C₄ alkyl), of which methyl esters are preferred. The fatty acids preferably comprise polyunsaturated fatty acids, having a chain length of C₁₂ to C₂₂, preferably C₁₃ to C₁₉.

A preferred mixture of (isotopically labelled) polyunsaturated fatty acids for the media of the invention is present in a lipid extract obtained from isotopically labelled biomass of *Rhodophyta, Cyanidiophyceae* (in most cases it is mentioned as belonging to Rhodophyta), *Chlorophyta*, *Cyanophyta*, *Diatoms*, *Phaeophyceae*, *Dinoflagelate*. *Dinophyta*.

The final concentration in the nutrient medium for the (isotopically labelled, polyunsaturated) fatty acids and/or their (methyl) esters are preferably from 2 mg/l to about 100 mg/l, more preferably from 10 to 30 mg/l, and most preferably 18 to 22 mg/l.

The steroids in the source of lipids are preferably sterols such as lanosterol, stigmasterol, sitisterol and cholesterol, whereby cholesterol is particularly preferred. Steroids may be obtained from lipid extracts by precipitation as described by Young and Britton (1993, Carotenoids in Photosynthesis. Chapman & Hall), and by Volkman, 2003 Appl. Microbiol. Biotechnol., 60(5): 495-506. The final concentration in the nutrient medium for the (isotopically labelled) steroids, preferably is from 2 to 10 mg/l, more preferably 5 to 7 mg/l.

The lipid soluble vitamins in the source of lipids preferably at least comprise vitamin E (alpha-tocopherol) and may further e.g. comprise vitamin A. The final concentration in the nutrient medium for the (isotopically labelled) alpha-tocopherol preferably is from about 0.5 mg/l to about 4 mg/l, more preferably, 1.5 to 3 mg/l. Sufficient quantities of vitamin A will be present in the lipid extracts.

Direct addition of lipids to the aqueous media of the invention is impractical due to their low solubility in water. The source of lipids is therefore preferably provided in the form of a suitable lipid emulsion, such as e.g. a microemulsions as described by Maoiella et al. (1988, BioTechnol. 6: 1406). Such a microemulsion comprises a lipid/organic solvent solution containing a small amount of emulsifier. Preferred, emulsifiers include an anionic surfactant, usually phospholipids, such as lecitin, or non-toxic, non-ionic polymeric detergents, such as e.g. polysorbate 20 or 80. A further possibility is to add the lipids dissolved in a water-miscible organic solvent such as e.g. dimethylformamide and a variety of alcohols (C₁-C₆ alcohols), ethanol being preferred. The stock-solution of the lipids in the water-miscible organic solvent is preferably such that the final concentration of the organic solvent is the nutrient medium is less than 0.1% (v/v), more preferably less than 0.05 or 0.01%, to avoid toxicity of the solvent towards the insect or mammalian cells. The skilled person will empirically determine the final concentration of organic solvent depending on the chosen solvent, the type cells to be grown and the like.

A preferred isotopically labelled source of lipids for a nutrient medium of the invention comprises per litre of medium: 20 mg of isotopically labelled lipid extract, 6 mg of isotopically labelled cholesterol, dissolved together in 1 ml ethanol.

Optionally protective agents may be incorporated into the nutrient medium of the invention. Protective agents are generally known in the art and are defined as non-toxic, water soluble components that functionally act to protect insect or mammalian cells from damage and death in agitated and sparged insect cell culture. Since their concentration (weight-volume) is very low (of 0.01 to about 1%) and they will usually not immediately be metabolised by the cells in culture, they may be added in unlabelled form. The protective agent may be added separately or may be combined with the source of lipids. The protective component preferably comprises block polymers of propylene oxide and ethylene oxide, and more preferably Pluronic polyols such as Pluronic F68 and F88 (BASF Wyandotte Corp.) Other suitable compounds that may serve as protective agents include e.g. hydroxy-ethylstarch, methylcellulose, carboxymethylcellulose, dextran sulphate, polypropylenglycol, alginic acid, Ficoll and polyvinylpyrrolidone.

To the optionally filtered/sterilised 1 ml of lipid component solution (described above) 10 ml of 10% of Pluronic F6S in water (optionally filtered/sterilised) is slowly added with agitation as by vortexing. The Pluronic polyols are commercially available from BASF Wyandotte corp. (101 Cherry Hill road, P.O. Box 181, Parsippany, N.J.07054, U.S.A.). A lipid component microemulsion is thereby formed. This lipid emulsion may then be added to the medium. The success of preparation of the lipid emulsion may be temperature dependent, preferably, therefore the emulsion is prepared at a temperatures in the range of 35-40° C. To facilitate in the preparation high-speed vortexing may be applied. Preferably, the source of lipids is filtered and (filter-) sterilised. Alternatively, the medium after being composed may be filter sterilised. Sterols are provided from the organic extract together with the other class of lipids.

Suitable sources of sterols are Cyanidiophyceae, red algae, other algae. A major portion of the free water-soluble isotopically labelled vitamins is provided via the isotopically labelled peptone component, typically by isotopically labelled yeastolate and isotopically labelled methylotrophs. Lipid-soluble vitamins are provided from the organic extract together with the other classes of lipids.

Furthermore, insect blood contains an unusually high level of free organic acids such as citrate, succinate, oxalate or malate at range of 0.1-35 mmol per insect (Grace, (1962), Nature, 195, 700, Vaughn, J.L., (1968) Curr. Top. Microbiol, Immunol. 42, 103). The Krebs cycle intermediates are good chelating agents and therefore play an important role in the cationic balance of the hemolymph. The cell culture media of this invention may be supplemented with one or more Krebs cycle intermediates and/or pyruvate that are preferably added up to a maximum concentration of 50 mg/l of a separate organic acid. Some media having reduced amounts or none of these components still support insect cell growth. (Gardiner & Stockdale, 1975). WO

01/98517 e.g. discloses that the most expensive of the free organic acids, such as fumarate, malate, succinate, ketoglutaric acid and hydroxyproline, can be eliminated entirely from the insect cells media with no ill effect and replaced by larger amounts (up to 250 mg/l for a separate vitamin) of vitamins as thiamin, riboflavin, niacin, vitamin B6, folic acid, vitamin B12, biotin, pantothenic acid, choline, para-aminobenzoic acid, inositol, sugars like glucose, and peptones.

Suitable sources of organic acids are described above and include Scyphozoan rhizostoma (releases organic acids in the cultural medium), green sulphur bacterium Chlorobium (when incubated in dark releases organic acids), purple non-sulphur bacteria Rhodospirillum rubrum (during anaerobic growth in dark). Organic acids are provided either from organic extract of biomass or separately as an extract of cultural broth.

The media may further be supplemented with labelled pyruvate at 60 - 90 mg/L, as e.g. obtainable from Isotec Inc.

Optionally free, purified isotopically labelled amino acids may be added to the media of the invention (in addition to the above described sources of amino acids. Free amino acids, organic acids and vitamins preparations may either be purified from the isotopically labelled natural sources or may be obtained commercially and are conventionally added to the basic basal medium independent of any amino acids and/or vitamins derived from the hydrolysates or yeastolate component of the complete medium

### Methods for producing isotopically labelled biomolecules

In another aspect the invention relates to methods for producing isotopically labelled biomolecules. Preferably the method is a method for producing a biomolecule, whereby 95% or more of the atoms in the biomolecule are isotopically labelled. The preferably comprises the steps of (a) growing a culture of mammalian or insect cells capable of producing the biomolecule under conditions conducive to the production of the biomolecule, in a nutrient medium produced in a method according to the invention, and (b) recovery of the biomolecule. Preferably the biomolecule is a polypeptide or a protein (both terms are used interchangeably herein). The methods of the invention are particularly suited for the production of isotopically labelled membrane proteins, including trans-membrane and extrinsic membrane proteins. However, the method of the invention is equally suitable for the production of soluble proteins or other non-membrane proteins. The protein preferably is a mammalian protein, more preferably a human protein.

In the method of the invention the protein is produced by culturing mammalian or insect cells that are capable of producing the protein in a nutrient medium wherein, for at least one of H, C or N, 95% or more of the atoms in substrates that are used by the cells for synthesis of the biomolecule, i.e. protein, in the nutrient medium are isotopically labelled. The protein may be an endogenous protein that is naturally produced by the cells in culture. However, typically the protein will be produced by recombinant means.

For this purpose a nucleotide sequence encoding the polypeptide of interest is expressed in suitable mammalian or host cells such as described in Ausubel et al., "Current Protocols in Molecular Biology", Greene Publishing and Wiley-Interscience, New York (1987) and in Sambrook and Russell (2001) "Molecular Cloning: A Laboratory Manual (3rd edition), Cold Spring Harbor Laboratory, Cold Spring Harbor Laboratory Press, New York.

The nucleotide sequence encoding the polypeptide of interest needs to be introduced into the host cells via an expression vector. The vector may be a replicative vector comprising on origin of replication (or autonomously replication sequence) that ensures multiplication of the vector in a suitable host for the vector. Alternatively the vector is capable of integrating into the host cell's genome, e.g. through homologous recombination or otherwise. Suitable expression vectors for mammalian cells are well known from Sambrook and Russell (2001, *supra).* Suitable vectors for insect cells are based on the well known Baculovirus (Merrington et al., 1997, Mol. BiotechnoL 8:283-97). The phrase "expression vector" generally refers to nucleotide sequences that are capable of affecting expression of a coding sequence of interest in hosts compatible with such nucleotide sequences. These expression vectors typically include at least suitable transcriptional (promoter) and translational initiation and termination regulatory sequences operably linked to the polypeptide-encoding segment. A DNA segment is "operably linked" when it is placed into a functional relationship with another DNA segment. In the expression vector the nucleotide sequence encoding the polypeptide of interest, is operably linked to a promoter capable of directing expression of the coding sequence in the host cell.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides know to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues.

The selection of an appropriate promoter sequence generally depends upon the host cell selected for the expression of the DNA segment. Examples of suitable mammalian promoter sequences are well known in the art (see, e.g. Sambrook and Russell, 2001, *supra).* Similarly suitable promoter sequences for use in insect cells are known in the art (Merrington et al., 1997 *supra).* Examples of workable combinations of cell lines and expression vectors are described in Sambrook and Russell (2001, *supra),* for mammalian cells in Wurm and Barnard (1999, Curr. Opin. In Biotechnology 10: 156-159), for insect cells in Massotte (2003, Biochim. Biophys. Acta. 1610: 77-89) and in Ikonomou et al. (2001, In Vitro Cell Dev. Biol.-Animal. 37: 549-559), and for yeast in Metzger et al. (1988, Nature 334: 31-36).

The method for producing a labelled polypeptide of invention thus comprises the step of culturing a host cell as defined above under conditions conducive to the expression of the polypeptide. Optionally the method may comprise recovery the polypeptide. The polypeptide may e.g. be recovered from the culture medium by standard protein Purification techniques, including a variety of chromatography methods known in the art per se (see below).

### Purification of labelled protein from cells/culture

Purification of (labelled) proteins from cell or culture medium is well known in the art. For purification of soluble proteins see e.g. Deutscher (1990, Methods in Enzymology, Vol. 182) and Yokoyama (2003, Current Opinin. Chemical Biology. 7: 39-43, and references therein). Methods for preparing large quantities of a given protein of sufficient purity for domain structure analysis are generally known to those of skill in the art. Although not all methods for protein purification are applicable to a given protein of interest, it is generally understood that the following methods represent preferred embodiments: affinity chromatography, ammonium sulphate precipitation, dialysis, FPLC, ion exchange chromatography, ultracentrifugation, etc. For a general review of protein purification see Burgess (1987, In: "Protein Purification", Oxender, et aL Eds., Protein Engineering, p.p. 71-82., Liss), Jacoby, Ed. Methods Enzymol 104: Part C (1984); Scopes, Protein Purification: Principles and practice (2-nd ed.), Springler-Verlag (1987).

Purification of membrane proteins is e.g. disclosed by Bosman et aL (2003, In: "Methods in Molecular Biology", volume 228: 73, Ed. Selinsky. Humana Press Inc., Totowa, NJ), Reeves et aL (2002, Proc. Natl. Acad. Sci USA 99: 13413-13418) and Eilers et aL (1999, Proc. Natl. Acad. Sci USA 96: 487-492).

WO 99/22019 discloses protein sample preparation for NMR. Solvent condition suitable for determining a biophysical property of protein is disclosed in WO 99/30165. Sample preparation of membrane proteins for NMR are reviewed by Sanders and Oxenoid (2000, Biochim. Biophys. Acta 1508 129-145, and references therein). Reconstitution of membrane proteins into lipid bicelles for NMR studies is described by Sanders et aL (1995 Biochem. 34: 4030-4040). NMR spectroscopy of large proteins is described in the US 6,198,281.
In a further aspect, the disclosure relates to a mammalian membrane protein whereby 95% or more of the atoms in the protein are isotopically labelled with an isotope selected from ¹⁵N, ¹³C, ²H, ¹⁵N and ¹³C, ¹⁵N and ²H, ¹³C and ²H, or ¹⁵N, ¹³C and ²H. Isotopic labelling of 95% or more of the atoms in the protein means that at least 95% or more of the atoms of a given element will be in the desired isotopic form, preferably more than about 98, 99, 99.5 or 99.9%. Preferably, the mammalian membrane protein is a protein whereby 20 - 100% of the hydrogen atoms in the protein are uniformly substituted with the isotope ²H. Preferably the membrane protein a human protein. A membrane protein is herein understood to be a protein comprises a polypeptide chain that has a length of at least 30,40,50, 70, or 100 amino acids, so as to distinguish the protein from a peptide that may be derived from a full-length protein.

### Method for determining three-dimensional structure

In a further aspect the invention relates to a method for obtaining structural information and/or information on the structure-function relation of a biomolecule. Preferably the method is a method for determining the three-dimensional structure (3-D structure) of a biomolecule. The method comprises producing an isotopically labelled biomolecule in insect or mammalian cells that are grown in a nutrient medium of the invention as described above. The isotopically labelled biomolecule, preferably a (transmembrane) protein, is than recovered and optionally purified from the cultured cells and/or medium, optionally further purified and subjected to spectroscopic analysis. Such spectroscopic analysis may be nuclear magnetic resonance ("NMR") spectroscopic analysis, Fourier Transformed Infra Red spectroscopic analysis and/or Raman spectroscopic analysis, to determine information about the structure, structure-function relation and preferably the three-dimensional structure of the biomolecule.

In a preferred method the biomolecule is a protein complexed to a second biomolecule. The second biomolecule may also be produced in a method according to the invention, whereby preferably 20 - 100% of the hydrogen atoms in the second biomolecule are uniformly substituted with the isotope ²H. The second biomolecule may be a protein.

Nuclear magnetic resonance ("NMR") spectroscopy, discovered by Felix Bloch and Edward Purcell in 1946, has evolved into an important technique with well known applications in chemistry, life sciences and molecular diagnostics including structural analysis of biomolecules such as proteins and nucleic acids (Wütrich, K., (1986) NMR of proteins and nucleic acids, Wiley, New York). Further technical advances in NMR, including e.g. pulse sequences and methodological developments, have increased the range of application for NMR in the study of protein-ligand interactions, forming the basis for the structure-based drug design (Marrasi and Opella, 1993, Curr. Opin. Struct. Biol. 8: 640; Watts et al., 1995, Mol. Membr. Biol. 12: 233). The diversity of applications of NMR to drug design and the possibly contributions of NMR to drug discovery process are reviewed by Roberts (2000; DDT, 5: 230). Today such strategies as SAR by NMR (structure-activity relationship by nuclear magnetic resonance) technique (Shuker et al., 1996, Science, 274: 1531), the "shapes screening" (Bemis, 1996, J. Med. Chem. 39: 2887) and NMR-Solve (structurally oriented library valency engineering) have wide application in drug discovery (Pellecchia, et al., (2002, Nature, 1: 218).

Uniform isotope labelling, selective labelling and segmental labelling are known labelling techniques that are applied in the NMR. Uniform isotope labelling of the protein, enables the assignment process through sequential assignment with multidimensional triple-resonance experiments and supports the collection of conformational constrains in *de novo* determination of protein structure (Kay, et al., 1997, Curr. Opin. Struct. Biol. 7: 722).

Selective labelling of individual amino acids or certain amino-acid types in proteins typically result in a marked simplification of the spectrum (Pellecia, et al.,2002, J. Biol. NMR 22: 165; Strauss, et al., 2003, J. Biomol. NMR 26: 367-372). Selective labelling can be accomplished by using a culture medium with full amino acid complement and replacing selected unlabelled amino acids by labelled ones with the desired stable isotope. Under typical conditions the amount of yeast plus algal autolysate may be reduced to 0.1 % (w/v) without significantly effecting growth or protein production, and this will dilute label incorporation by maximally 5%. Substitution of some amino acids however, in particular Glu and Gln, will lead to scrambling of label into other (non-essential) amino acids.

A variant approach is "segmental labelling" in which discrete segments of the polypeptide chain are uniformly labelled, whereas other are not (Xu et al., 1999, Proc. Natl. Acad. Sci. USA. 96: 388).

For NMR analysis of membrane proteins Solid State NMR (SS NMR) (Ernst, R.R., et al., 1987, in: Principles of Nuclear Magnetic Resonance in One and Two Dimensions, Oxford, Claredon press; Mehring, M., 1983, in: Principles of High Resolution NMR in Solids, Springer Verlag Berlin) is the most suitable technique. SS NMR inter alia allows to perform routine assays of ligand-protein interactions for ligand when bound to their receptor target in the membrane (de Groot, 2000, Curr. Opin. Struct Biol., 10: 593). This is of particular interest for the pharmaceutical industry integral membrane proteins as is illustrated by the fact that 60% of all prescription drugs are targeted against membrane proteins and is high-lighted by the family of G-protein-coupled coupled receptors (Essen, L-O., (2002) Gene Funct. Dis., 3, 39). In SS NMR technique, there are two approaches that can be used to deal with the anisotropic interactions, which lead to NMR spectral broadening and permit molecular structural information to be extracted. One is to exploit the spectral anisotropy in oriented samples to give molecular orientations in static samples (Griffin, R.G., (1998) Nat. Struct. Biol, 5, 508., Opella, S.J. et al., (1999) Nat. Struct. Biol., 6, 374), and the second is to use random dispersions in magnetic angle spinning (MAS) NMR (Andrew, E.R, et al., (1958) Nature 182, 1659), in which orientational information may be lost but can be regained from analysis of spinning side-bands. In addition, dipolar couplings can be refocused to yield precise distance constrains, as well as chemical shift information to define local environmental characteristics.

MAS-based methods may be successfully applied to determine internuclear distances or torsion angles well beyond the resolution obtainable in X-ray crystallography (Thomson, L.K.(2002) Curr. Opin. Struct. Biol., 12, 661). A prerequisite for structure determination of uniformly labelled peptides and proteins are hetero- and homonuclear assignment techniques. For both aspects, a variety of pulse schemes is available in the art (Baldus., et al., (1998) Mol Phys., 95, 1197; and Bennett, 1994, In: "NMR Basic Principles and Progress", Springer-Verlag Berlin., 33: 1). Recent examples of protein structures determined by MAS NMR (Castellani, et al., (2002) Nature, 420 (7): 98; and Pauli., et al., (2000, J. Magn. Res., 143: 411) is the α-spectrin Src-homology 3 (SH3) domain containing 62 amino acid residues where an almost complete ¹³C and ¹⁵N resonance assignment for a micro-crystalline preparation of the protein formed the basis for the extraction of a set of distance constraints. Many of the resonances of membrane spanning parts of the light-harvesting complex LH2 from purple non-sulphur photosynthetic bacterium of *Rhodopseudomonas acidophila* 10050 were reported (Egorova-Zachemyuk., et al., (2001) J. Biomol. NMR, 19, 243). Partial assignments were also reported for ubiquitin (Hong, (1999) J. Biomol. NMR, 15: 1; Pellegrini, M., (1999) Biopolymers, 51, 208) and BPTI (McDermott., et al., (2000) J. Biomol. NMR., 16. 209). The number of residues in these peptides also compares favourably to membrane-spanning or surface-bound peptides studied recently in oriented lipid bilayers (Opella, S.J., et al., (1999) Nat. Struct Biol, 6, 374). It can thus be inferred that MAS-based correlation techniques could also be used to study entire membrane-protein topologies or subsections thereof.

Generally, with further combination of refined isotope-labelling schemes and further improvements in NMR instrumentation, the size and complexity of NMR-accessible receptor/ligand systems will be widened. In addition, with the parallel developments in the expression of membrane proteins NMR holds considerable promise for the future. Solid state NMR allows direct examination of the protein in a non-crystalline environment, a direct examination of a protein in the membrane, either as a single species (reconstituted in defined lipids) or in a heterogeneous environment with other proteins and lipids (natural membranes), but importantly under non-perturbing conditions, a detailed description of orientational constrains with respect to the membrane to be resolved, an identification and a resolution of specific parts of protein, especially when these are at vital ligand binding sites, a study of a protein with no molecular weight limit (in this case assignment strategies should be further developed), a definition of pharmacophores for ligands for membrane-bound receptors to be defined from chemical shift data for bound ligands. To date, however, only a few membrane proteins or peptides have been studies using SS NMR. Both structural methods such as 3D-crystallisation and X-ray crystallography or, alternatively, NMR-spectroscopy require at least about 10 mg of highly purified membrane proteins.

### Description of the Figures

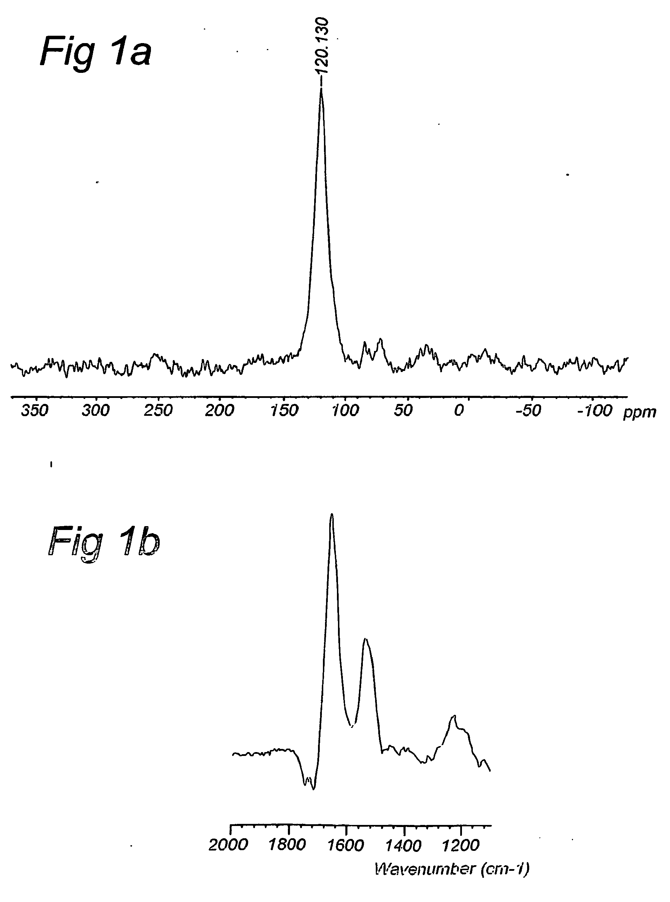

Figure 1A. ¹⁵N 1D MAS SSNMR spectrum of stable isotopically labeled membrane preparation purified from Sf9 cells recorded at 750 MHz with a MAS frequency of 10 kHz at a temperature of 200 K. This spectrum was obtained after 5000 scans. During acquisition, TPPM (Bennett, A.E., et al J. Chem. Phys., 1955, 103, 6951) proton decoupling at 80 kHz rf field strength was applied. Cross polarization (CP) ¹H-¹⁵N mixing time of 2 ms was applied. Major resonance at 120 ppm represents the backbone peptide group nitrogens. The other smaller resonances represent amino acid- residues side chains of the protein. Gly nitrogen resonance at 34.3 ppm has been used as a reference.
Figure 1B. FTIR difference spectrum demonstrating ¹⁵N incorporation in SF9 cellular membranes. The positive/negative peak combination at 1580/1535 (on the sloping edge of the 1620 peak) represents the shift of the Amide II vibration upon backbone peptide bond labeling.

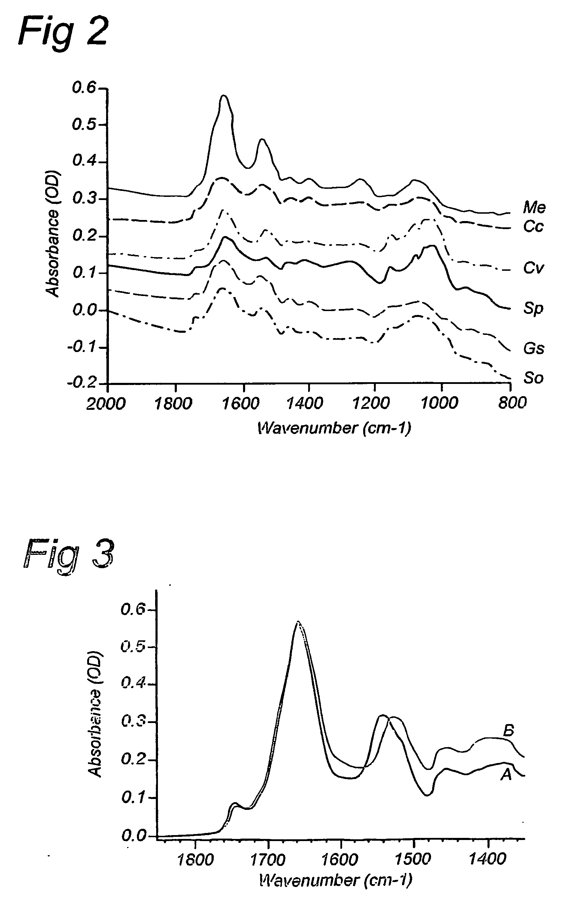

Figure 2. Rapid compositional analysis of biomass by transmission Fourier-transform infrared (FTIR) spectroscopy. Protein (1700-1500 cm⁻¹) and carbohydrate (1200-1000 cm⁻¹) vibrational bands can be easily monitored for reproducibility of the (labelled) biomass composition. Me - *Metyalobacterium extorquens;* Cc - *Cyanidium caldarium;* Cv - ***Chlorella** viridus;* Sp - *Spirulina platensis;* Gs - *Galdieria sulphuraria;* So - *Scenedesmus obliquus.*
Figure 3. Monitoring of ¹⁵N stable isotope labelling with FTIR from the shift of the Amide II vibration (1542 cm⁻¹). The figure shows an expanded part of an infrared spectrum of *Hansenula polymorpha* yeast biomass cultured on an unlabelled medium (A) and on a ¹⁵N stable isotope labelled medium (B). The spectra are plotted after baseline correction and scaling using the strongest peak (1658 cm⁻¹) as a reference. ¹⁵N label incorporation was 99%.

### Examples

### Example 1. Production of stable isotope labelled biomass, hydrolysate and extracts therefrom (yeast as a source)

### 1.1 Yeast extracts comprising amino acid and sugar source obtained from Saccharomyces cerevisiae

*Saccharomyces cerevisiae* (ATCC 13057) was grown in a medium containing (g/l): ¹³C uniformly labelled glucose - 15 g/l, labelled KH2PO4 - 2 g, ¹⁵NH₄Cl-1.5 g, MgSO₄ x 7 H₂O - 0.5 g, CaCl₂ x 6H₂O - 0.25 g., FeSO₄ x 6H₂O - 0.036, ZnSO₄ x 7H₂O - 0.001 g, MnCl₂ x 4H₂O - 0.001 g, CoCl₂ x 6H₂0 - 0.001 g. Vitamins were added in the same concentrations as described by Heine, W., et al in Stable Isotopes in Pediatric Nutritional and Metabolic Research (1990) (Eds., T.E. Chapman, R. Berger., D.J. Reijngoud, and A. Okken, Intercept Ltd., p. 84. The yeast were cultured as shaking culture in 10-1 conical flasks containing 2.5 1 of the sterilised at 120° C at 20 min medium at pH 4.5 adjusted with NaOH. To this flask 100 ml of seed culture was transferred. Seed culture was obtained by shaking in a flask at 27 °C for 18 hours. Yeasts were grown with stirring 800 rpm at 27°C for 20 h. The yeast cells were collected by centrifugation at 5,000 g for 10 min at 4 °C and washed twice with water. To the washed cells water was added to prepare 500 ml of yeast slurry of about 100 mg/ml of dry weight basis.

Half of the volume of the slurry was lyophilised and used for the preparation of yeast hydrolysate. Yeast hydrolysate was prepared as described in Example 5.1.

Another half of the slurry was used for autolysis. It was squeezed under slight pressure through a curved syringe kept at 65 °C (ID 2-3 mm) keeping the transit time below 20 seconds, and then collected in a flask cooled on ice. Then the slurry was kept in an oil thermostat at 45 °C for 4 hours while pH was adjusted to 3.0 by drop wise addition or 20 % NaOH. After the autolysis pH was adjusted to 6.5 with 2N HCl and autolysate was warmed up to 80 °C. Then the autolysate was centrifuged and precipitate was washed twice with water. The combined supernatant of 400 ml, comprising autolysate was then filtrated through a 10,000 cut-off filter and lyophilised yielding 15 g of yeast extract.

Chemical analysis of the yeast extract was performed as described in T. Hemawan, G. Fleet., J. Ind. Microbiology (1995) 14, 440. Typically based on initial cell dry weight, the soluble autolysate of *Saccharomyces cerevisiae* consisted of carbohydrates (4-8%), protein (12-15%), organic acids (3-5%), free amino acids (10-12%), oligopeptides (30-34 %), nucleic acid products (3-5%), lipids (2-3%) and other physiologically active compounds.

### 1.2 Alternative protocol for yeast autolysis

To a yeast slurry obtained as described in Example 1.1 2 volume percent of ethanol was added and autolysis was performed at 50°C during 12 hours. The yeast autolysate was then treated with ultrasound tip-sonicator for 6 minutes followed by centrifugation at 5000 g 10 min. The residue was washed twice with the distilled water and centrifuged during 15 min. The combined supernatants were filtrated through a 10,000 cut-off membrane filter and lyophilised. As a result mixtures of amino acids, peptides, carbohydrates, nucleic components, organic acids and other components were obtained. This procedure allows increase of a free amino acid content up to 80 %.

### 1.3 Yeast extract of Pichia pastoris

*Picha pastoris* NRRL-Y-11430 was grown on ¹³C labelled glycerol and glucose as a carbon energy substrate and (¹⁵NH₄)₂SO₄ as a nitrogen source as described in M.J. Wood and E.A. Komives., (1999) J. Biomol. NNM 13, 149 or on a ¹³C methanol as a carbon and energy source. Yeast extracts (acid hydrolysate and autolysates) were obtained using the same methods as described in Examples 1.1, 1.2 and 5.1.

### 1.4 Yeast extract of Hansenula polymorpha

*Hansenula polymorpha* CBS 4732 (syn. ATCC 34438, NRRL Y-5445) was growing in a methanol-limited flow-controlled continuous culture on a medium similar to as described by Y. Laroche., et al. (1994) Biotechnology 12: 1119., Th. Egli, et al., Arch. Microbiol (1982) 131, 8., and Van Dijken, et al., (1976) Arch. Microbiol. 111: 137, where ¹⁵N labelled salt was used as a nitrogen source and uniformly ¹³C labelled glucose and ¹³C labelled methanol were used as a carbon and energy source. Cultivation was performed at 35° C and at pH of 5.0. Yeast extract (acid hydrolysate and antolysates) were obtained using the same methods as described in Examples 1.1, 1.2 and 5.1 accordingly.

### 1.5 large scale production of yeast biomass

A 3 liter fermenter (BioFlo3000, New Brunswick Scientific) was used for yeast culture in order to have full control over the system (temperature, pH, oxygen-,nitrogen-,air-supply, dissolved oxygen). An optimal yeast growth was achieved with the aeration of 25% oxygen and 75% nitrogen with a dissolved oxygen level of 35%. Yeast were growing at 30°C using 2 liters of either modified Heine's medium or modified Invitrogen medium wherein glycerol was replaced by glucose. The agitation speed was 500 rpm. In both cases all carbon and nitrogen sources were replaced with the isotopically labeled analogues. Glucose was either added in batch or a fed-batch culture was applied.

### Example 2. Production of stable isotope labelled biomass, hydrolysate and lipid

### extracts therefrom (algae as a source)

### 2.1. Production of stable isotope labelled Cyanidium biomass

*Cyanidium caldarium* (SAG 16.91) and *Galdieria sulphuraria* (SAG 17.91) were each grown autotrophically at 25° C in 51 flasks with magnetically driven stirring bars in constant temperature water bath at a constant pH of 2 and harvested during the exponential growth phase. Medium composition for ¹³C, ¹⁵N double-labelled cultures of *Cyanidium caldarium* and of *Galdieria sulphuraria* contains per litre 1.5 g (¹⁵NH₄)₂ SO₄, 0.3 g Mg SO₄ x 7 H₂O, 0.3 g KH₂PO₄, 0.02 g CaCl₂ x 2 H₂O, 1.5 ml of an Fe-EDTA solution (Fe-EDTA solution was prepared by adding of 0.690 g of FeSO₄ and 0.930 g of EDTA to a volume up to 100 ml of distilled water and boiling of solution), and 2 ml of a trace element solution that was prepared separately (see below). The pH of the medium was adjusted to a value of pH 1.8 with 1N H₂SO₄. Trace element solution contains per litre: 2.86 g H₃BO₃, 1.82 g MnCl₂, 0.22 g ZnSO₄ x 7 H₂O, 0.130 g Na₂MoO₄ x 2H₂O, 80 mg CuSO₄ x 5 H₂O, 40 mg NaVO₃ x 4 H₂O, and 40 mg CoCl₂ x 6 H₂O.

A continuous light source of 8000 lux was supplied by 15-W fluorescent lamps and under a concentration of ¹³CO₂ up to 5 %. Growth of the algal suspension was assayed by absorbance measurements at 550 nm. When growth reached exponential phase (usually 5 days) (0.64 OD) the cells were harvested with Beckman centrifuge at 5000 rpm for 10 minute and the pellets were cashed with distilled water to remove traces of soluble nutrients. The resulting paste was frozen and lyophilised. The lyophilised cells were used for preparation of lipid extracts and hydrolysate.

### 2.3. Production of stable isotopes labelled green algal biomass (Scenedesmus obliquuis)

¹³C-, ¹⁵N-double-labelled algal biomass of *Scenedesmus obliquus* was produced as described by Patzelt, H., et al., 1999 Phytochemistry 50: 215-217. *Scenedesmus obliquus* strain 276/3C (obtained from Culture Collection of Algae and Protozoa (CCAP), Institute of Freshwater ecology, Ambleside, UK and was grown autotrophically at 30° at a pH of 6.5 and harvested during the exponential growth phase. Medium composition for ¹³C, ¹⁵N double-labelled cultures of *Scenedesmus obliquus* strain contains per litre 0.18 g Na₂HPO₄ , x 2H₂0, 0.48 g NaH₂PO₄, 0.46 NaCl, 0.25 g MgSO₄ x 7H₂0, 6 mg CaCl₂ x 2 H₂O, 6.5 g FeSO₄ x 7H₂0, 8 mg EDTA, 2.9 mg H₃BO₃ 1.8 mg MnCl₂x 4H₂0, 0.22 g ZnSO₄ x 7 H₂O, 0.25 mg Na₂MoO₄ x 2H₂O, 0.08 mg CuSO₄ x 5 H₂O, 0.08 mg CoCl₂ x 6 H₂O and 0.81 g K¹⁵NO₃. The pH of the medium was 6.5. The medium was prepared in distilled water that was extensively degassed to remove any dissolved CO₂. ¹³CO₂ was supplied to the culture system as NaH¹³CO₃ in carbonate buffer.

For precultures two reservoirs were used. A lower reservoir contained 2M carbonate (Na₂¹³CO₃/NaH¹³CO) buffer, pH 7.5 (100 ml), an upper flask contains inoculate. After inoculation the flasks were sealed and shaken at 60 rpm at 30 °C under constant illumination for 10 days. A continuous light source of 2000 lux was supplied by 15-W fluorescent lamps (cool white). For the buffer preparation 11 of 1.2 M NaOH was bubbled with 161 of ¹³CO₂ until pH was 9.3, 50 mg of phenolphatalein was added for monitoring of the pH.

For preparative fermentations a closed system was used, that consists from the 10 2.5 1 Fernbach flasks (11 of medium in the flask with 5 ml of inoculate), carbonate buffer and a gas pump The air is circulating in the circle so that the cell cultures will be followed with the ¹³CO₂-enriched air. The algae were growing 10-14 days. A steady current of 5% ¹³CO₂ in compressed air was blown over Fembach flasks. Growth of the algal suspension was assayed by absorbance measurements at 685 nm. When growth reached exponential phase in 17 days (1.0 OD) the cells were harvested at a temperature of 4 °C with Beckman centrifuge at 5000 rpm for 10 minutes and the pellets were washes with distilled water to remove traces of soluble nutrients. Culture broth was used for the further growth cycle (after the filtration. The resulting paste was frozen and lyophilised. The freeze-drying is essential since the presence of water can cause the breakdown of chlorophylls via chlorophyllase. The yield of the biomass was 2 g of lyophilised cells per 1 culture. Lyophilised cells were used for preparation of lipid extracts and hydrolysate.

Upscaling of the growth of algae to a 201 bioreactor gave similar results.

### Example 3. Production of stable isotope labelled biomass, hydrolysate and extracts therefrom (methylotrophic bacterium as a source)

### 3.1. Production of biomass from a methylotrophic bacterium

An obligate methylotroph *Methylobacillus flagellatus* (ATCC 51484, VKM B-1610, DSM 6875) was grown at 30° C at pH 6.8 on a ¹³C - labelled methanol as a carbon source and ¹⁵NH₄Cl as a nitrogen source on the ATCC medium 784 AMS without agar. The concentration of ¹³C labelled methanol was 1%. Cells were growing in a 101 fermenter and harvested after 3 days of growing. The yield of the biomass was 53% calculated on ¹³C-methanol. The biomass was lyophilised. The yield of protein was 75% calculated on dry biomass. The lyophilised cells were used for preparation of lipid extracts and hydrolysate.

### Example 4. Extraction of stable isotope labelled lipids

### 4.1. Extraction of algal biomass (Procedure 1)

Lyophilised cells (10 g) of *Cyallidium caldrium* were homogenised in dichloromethane by sonication during 30 min on ice (cooling is achieved by ice with salt) by alternating 50% power for 30 sec and transferred into a Soxhlet extraction apparatus. Extraction was continued for 30 hours and then continued for another 24 hours with 90 % aqueous methanol until the extract becomes colourless. The combined extract was evaporated on a rotary evaporator under nitrogen and separated into non-lipid and lipid fractions by Sephadex chromatography Wuthier, RE., (1966) J. Lipid. Res, 7: 558-561. The lipid fraction was evaporated and dissolved in n-hexane-diethyl ether, followed by chromatography on a silicic acid column as described by Kleinschmidt, M.G. and McMahon, V.A. (1970, Plant physiol, 46: 286-289). Typical lipids were waves, hydrocarbons, sterol esters, triglycerides, sterols, free fatty acid, monoglycerides, diglycerides, glycolipids, sulpholipids, and phospholipids. In total 150 mg of lipids could be isolated from 1 g of the biomass. The major lipids of *Cyanidium caldarium* are mono- and- digalactosyldiglyseride and sulpholipid, lecitin, phosphatydil glycerol, phosphatidyl inositol, phosphatidyl ethanolamine.

Alternatively the purification of fatty acids and sterols could be done from the combined soxhlet extracts (total amount of 160 mg of lipid extract) obtained from 1 g of dry biomass of *Cyanidium caldarium* as is described by Ikan, R, Seckbach, J. (1972) Phytochemistry, 11: 1077-1082. As a result 32 mg of free fatty acids, and 39 mg of fatty from saponification of neutral fraction were obtained. Sterols were isolated from the unsaponifiable fraction (Ikah, *supra*) and their amount was 2 mg.

This procedure was used for lipid extraction from blue-green, red and cyanidiophyceae algae.

### 4.2. Extraction of algal biomass (Procedure 2)

To an amount of 10 grams of freeze-dried ¹³C, ¹⁵N labelled biomass of *Scenedesmus obliquus* 100 ml of hexane was added and the samples were sonicated in a tip-sonicater at - 4 °C (cooling is achieved by ice with salt) for at least 60 minutes. Solvent was removed via filtration and the rest of the biomass was extracted with 100 ml of acetone followed by the same procedures as has been described above in case of hexane extraction. Finally, the rest of the biomass was extracted with 100 ml of dichloromethane/methanol mixture (2:1 v/v). Filtrates were separately evaporated to dryness under nitrogen and stored at - 80 ° C. Acetone extract comprises a source of uniformly labelled pigments that could be further isolated in a pure form and used for example for structure-function studies of pigment-protein interaction in photosynthesis. In case of blue-green, red and cyanidiophyceae algae an extra step was used comprising the extraction with 90% aqueous MeOH. The extracts were handled as described above. The delipidated biomass (8 g dry weight) was used then for the preparation of hydrolysate.

This procedure was used for lipid extraction from all of the sources.

### 4.4. Extraction of red algae biomass (Porphyridium)

*Porphyridium purpureum* (=*Porphyridium cruentum*) (ATCC 50161) was grown on a ATCC 1495 ASW medium, but with K¹⁵NO3 and NaH¹³CO₃ instead of KNO3 and NaHCO₃ and with 54 g of NaCl per litre medium at 25°C and pH of 7.6. The algal cultures were illuminated continuously by fluorescence lamps at light intensity of 25 W/m². Lipids were extracted according to procedure 1 (Example 4.2) and determined according the method described in Ohia, et al., 1993, Botanic marina, 36: 1043-107. Using such a conditions for growing a high concentration of C_{20:4 (n-6)} and C_{20:5 (n-3)}, namely 8.2 mg dry weight and 14.3 mg dry weight per 1 g dry biomass was achieved.

### 4.5. Extraction of methylotrophic biomass (Methylobacillus)

The biomass of *Methylobacillus flagellatus* (10 g) was extracted as described in 4.3. The delipidated biomass (8.5 g) was used then for the preparation of hydrolysate as described in Examples 5.1 and 5.2.

### 4.6. Transesterification of lipids for analysis

Extracted lipids from *Galdieria sulphuraria* are transesterified to form fatty acids methyl esters, allowing identification and quantification of fatty acids using gas-chromatographic technique as described by Lewis et al. (2000, J. Microbiol. Meth. 43: 107-116). Freeze-dried cells were weighted (5g) to which a fresh solution of the transesterification reaction mix (10/1/1 v/v/v) was added as described. Cells were suspended in this solution by vortex mixing and immediately placed at 90°C for transesterification for 60 min. Then the transesterification reaction tubes were cooled down, water (1 ml) was then added to each tube and the fatty acids methyl ethers were extracted (hexane/chlorophorm, 4/1 v/v 3x4 ml). The chemical identity of the organic solvent extracts was monitored by TLC (e.g. silica gel, hexane/EtOAc, 5:1) and HPLC (C18, grad. Me₂CO-H₂O (1:4) to Me₂CO). Isotope composition was determined by EI-MS (70 eV) and/or infrared spectroscopy.

### Example 5 Hydrolysis of biomass

### 5.1 Acidic hydrolysis of isotopically labelled biomass

To an amount of gram of biomass free of lipids and pigments (Example 4.2) thus obtained and placed in the 240 ml flask, 15 ml of 6 N HCl and 0.6 ml of thioglycolic acid as a reducing agent (to prevent destruction of histidine and tryptophane) was added. Hydrolysis was performed under the argon at 110 °C (on the oil bath) for 24 h. 15 ml of distilled water was added to the hydrolysate that was cooled on ice, followed by filtration through the glass P3 filter. After this the hydrolysate was divided into two portions and lyophilized it two round bottom flasks. Then an amount of 10 ml of distilled water was added to each of the hydrolysates while one of them was neutralised with 30% NaOH, the other one with 30% ¹⁵NH₄OH. Half of the each of the portion was passed through the coarse-pored active charcoal of 20-100 Å. (2 cm high column), prepared using a glass P3 filter. As a result four hydrolysates were obtained that were further lyophilised. Lyophilisation lead to a white solid powder (amino acid/sugars mixture) for the hydrolysates passed through charcoal and to hazelnut powder for the hydrolysates not treated with charcoal. The hydrolysates (4 different) were further ground to formulate a homogeneous powder.

Alternatively hydrolysates were prepared without a "charcoal step" and for example in case of *Scenedesmus obliquus* (as well as *Cyanidium caldarium*) an amount of 0.6 g of the hydrolysate neutralised with ¹⁵NH₄OH and 0.7 g of the hydrolysate neutralised with NaOH has been obtained from an amount of 1 g of delipidised biomass. The upscaled procedure for hydrolysing of an amount of 20 g of delipidised biomass was used in most cases. The hydrolysates neutralised with ¹⁵NH₄OH were preferentially used for the formulation of insect and mammalian cell culture medium (see Example 6).

Amino acid composition (as determined based on RP HPLC FMOC- derivatisation method) of e.g. hydrolysate of *Cyanidium caldarium* neutralised with ¹⁵NH₄OH was Ala-8.8%, Arg-5.1 %, Asp - 5.2%, Glu - 9.5%, Gly- 7.3 %, His - 0.8%, Ile- 6.7 %, Leu - 6.5%, Lys- 5.6%, Met- 3.2%, Phe - 6.3%, Pro- 7.6%, Cys - 0.8 %, Ser- 11%, Trp- 0.8 %, Thr-6.1%, Tyr-1.2%, Val- 7.5%.

The hydrolysates were further used either for direct composition of insect and mammalian cell culture media or for the preparation of pure amino acids (by ion exchange chromatography (Crespi & Katz, 1972, Methods Enzymology 26, 627) or reversed-phase chromatography (Egorova, et al. T.A., (1995) J. Chromat, Biomedical Application 665: 53-62).

Alternatively the hydrolysate would be ultra filtered through a cassette membrane, cut-off 8 K, and a sterile 0.2 micron Durapore filter. The sterilised hydrolysate could be stored for 6 months at 4° C.

An improvement of the acid hydrolysis method is aimed at preparing hydrolysates having a low(er) salt content. In this improved procedure the lyophylisation step was performed twice: first, immediately after the hydrolysis (to remove also most of the HCl), and second, after the neutralisation either with sodium-or with ¹⁵N labelled ammonium-hydroxide.

### 5.2. Enzymatic hydrolysis of stable isotope labelled biomass

The delipidised biomass of *Methylobacillus flagellatus* was enzymatically hydrolysed similar to as described by Troxler, et al. (1975) 14(2): 268-274, immobilised Pronase (Fluka, Switzerland) was prepared according to the Pierce instructions and used for the enzymatic hydrolysis. The digest was lyophilised and used as a component of the medium for mammalian and insect cells.

### 5.3 Autolysis of stable isotope labelled algal biomass

The procedure for making yeast autolytic extracts, as described above in Examples 1.1 and 1.2, was applied for obtaining autolysates from algal biomass. Stable isotope labelled biomass from various algae was thus autolysed successfully, e.g. *Cyanidium caldarium* and *Galdieria sulphuraria.* Yield of autolysates was 35-45% calculated on a dry weight of the biomass. The remainder of the biomass material was subjected to acid hydrolysis (as described in Example 5.1) in order to obtain algal hydrolysates and to efficiently use the labelled biomass.

### Example 6 Composition of isotopically labelled nutrient media

### 6.1.1 Composition of stable isotope labelled nutrient medium for insect cells I

Composition of medium with respect to microelements, salts and vitamins was based on the IPL-41 formulation (Weiss., et al., 1981, In Vitro, 17: 495-502), except that NaH¹³CO₃ and sodium molibdate were used. Other components of the medium were added in the concentration given in the Table below.

| Components | Grams/Litre |
|---|---|
| ¹³C, ¹⁵N uniformly stable isotope labelled autolysate from algae (*Cyanidium caldarium*) | 5 |
| ¹³C, ¹⁵N uniformly stable isotope labelled hydrolysate from methylotrophs (*Methylobacillus flagellatus*) | 6 |
| ¹³C, ¹⁵N uniformly stable isotope labelled yeast autolysate (*Saccharomyces cereviciae)* | 5 |
| ¹³C uniformly stable isotope labelled glucose | 2 |
| ¹³C, ¹⁵N uniformly stable isotope labelled lipid extract (*Galdieria sulphuraria*) | 0.25 |
| Pluronic F-68 | 1 |

Lipid extract was added to the medium as an ethanolic solution that was prepared in a concentration of 50 mg of lipid extract (Example) per 1 ml of ethanol. Ethanol lipid solution was added in a concentration 5ml/l culture medium.

The hydrolysates were analysed by amino acid analysis (RP-HPLC as FMOC derivatives) and deficient amino acids (according to the IPL-formulation) such as ¹³C, ¹⁵N labelled cysteine were added separately to the medium in a concentration 80 mg/l.

pH was adjusted to 6.2, osmolarity is adjusted to 340 mOsmol kg-1 (NaCl). The complete medium was sterilised through a 0.22 micron filter and stored at 4 °C.

With 5-10 passages the Sf9 cell line was adapted to growth in isotopically labelled medium according to (Bosman, et al. in Method Mol. Biology V. 228, Ed. Selinsky, Humana Press Inc). Insect cells are cultured at a temperature of 27 °C and do not require CO₂ supplementation. Doubling time of adapted cells was 26-28 hours which is comparable to other commercially available serum-free media (S3777, Sigma., X-Cell 420 JRH Biosciences, HyQ SFX-Insect, Hyclone). Maximum cell density achieved was 4-6 x 10⁶ cells/ml.

### 6.1.2 Composition of stable isotope labelled nutrient medium for insect cells II

Composition of medium with respect to microelements, salts and vitamins was based on the IPL-41 formulation (Weiss et al., 1981, In Vitro, 17: 495-502), except that NaH¹³CO₃ and sodium molybdate were used. Other components of the medium were added in the concentration given in the Table below.

| Components | Grams/Litre |
|---|---|
| ¹³C, ¹⁵N uniformly stable isotope labelled autolysate from algae (*Cyanidium caldarium*) | 4 |
| ¹³C, ¹⁵N uniformly stable isotope labelled yeast autolysate (*Hansenula polymorpha*) | 8 |
| ¹³C uniformly stable isotope labelled glucose | 2 |
| ¹³C, ¹⁵N uniformly stable isotope labelled lipid extract (*Galdieria sulphuraria*) | 0.25 |
| Pluronic F-68 | 1 |

Lipid extract was added to the medium as an ethanolic solution that was prepared in a concentration of 50 mg of lipid extract per 1 ml of ethanol. Ethanol lipid solution was added in a concentration 5ml/l culture medium. Note that addition of lipid extract in this example is optional and the concentration used increased cell growth by no more than up to 10%. Without lipid extract cells were also growing and producing protein. The hydrolysates were analysed by amino acid analysis (RP-HPLC as FMOC derivatives) and deficient amino acids (with respect to the standard IPL41-formulation) such as ¹³C, ¹⁵N labelled cysteine and glutamine were added separately to the medium in a concentration 80 mg/l. pH was adjusted to 6.2, osmolarity is adjusted to 340 mOsmol kg-1 (NaCl). The complete medium was sterilised through a 0.22 micron filter and stored at 4 °C. Within 5-10 passages the Sf9 cell line was adapted to growth in isotopically labelled medium according to (Bosman, et al. in Method Mol. Biology V. 228, Ed. Selinsky, Humana Press Inc). Insect cells are cultured at a temperature of 27 °C and do not require CO₂ supplementation. Doubling time of adapted cells was 26-28 hours which is comparable to other commercially available serum-free media (S3777, Sigma, X-Cell 420 JRH Biosciences, HyQ SFX-Insect, Hyclone). Maximum cell density achieved was 4-6 x 10⁶ cells/ml.

### 6.1.3.Influence of the concentration of various components on insect media

In total biomass from more than 10 different microorganisms were tested, more than 100 different hydrolysates and autolysates were prepared from these biomass preperations and more than 10 different lipid extracts per microorganism biomass were tested. From these test the following general observations were made.

Yeast autolysates in general are incorporated at a concentration in the range of 0.1- 1.6% (w/v). Autolysates based on *Pichia pastoris* biomass grown on commercial Invitrogen media in which glycerol is replaced by glucose showed the best reproducibility and efficiency with respect to maintaining of insect cell growth with an optimal concentration for the autolysate of in the range of 0.1 - 0.3%, 0.2% was optimal (w/v). Autolysates based on *Hansenula polymorpha* biomass grown on a modified Heine's medium (with lactic acid omitted) showed the best reproducibility, efficiency with respect to insect cell growth with an optimal concentration in the range of 0.4%-0.8% (w/v). Yeast autolysates are usually effective with addition of small quantities of hydrolysates (for additional amino acid) or even only a few selected amino acids. Using our IPL-41 based media and cell lines on glutamine was requited in addition to yeast autolysate.

Algal autolysates in general are incorporated at a concentration in the range of 0.1 - 0.8 % (w/v). The optimal concentration may depend on the algal source, e.g for *Galdieria sulphuraria* the most effective concentration was 0.1 %, but for *Gyanidium caldarium* this was 0.4% (w/v). Again, also algal autolysates are usually effective with addition of small quantities of hydrolysates (for additional amino acids) or even only a few selected amino acids. In our hands only glutamine was required in addition to the algal autolysate. In contrast, algal hydrolysates are not very effective as single component (concentrations in the range of 0.1- 0.8% (w/v) have been tested) and need to be supplemented with more than a few selected amino acids.

Bacterial hydrolysates are generally incorporated at concentrations in the range of 0.1-0.4 % (w/v). Bacterial autolysates are generally in corporated in the range of 0.1-0.6 % (w/v).

Most effective in maintaining insect cell growth and protein production is a combination of autolysates based on a red algae, preferably *Cyanidium caldarium* or *Galdieria sulphuraria* and a yeast autolysate, preferably *Hansenula polymorpha* (see example 6.1.2). Effectivity of e.g. a medium based on an autolysate obtained from biomass of *Galdieria sulphuraria* could be increased by more than 70% when 0.8% of *Hansenula polymorpha* autolysate is added to the medium and/or effectiveness of the medium based on autolysates obtained from *Hansenula polymorpha* biomass could be increased for more than 60% % when 0.1% of the *Galdieria sulphuraria* autolysate is added.

### 6.2.1 Composition of isotope labelled nutrient medium for mammalian cells

Composition of medium with respect to microelements, salts and vitamins was based on the DMEM formulation (see e.g. JRH Biosciences, Cell culture and services), except that carbon and nitrogen containing salts were replaced by stable-isotope labelled ones. Other components of the medium were added in the concentration given in the Table below.

| Components | Grams/Litre |
|---|---|
| ¹³C, ¹⁵N uniformly stable isotope labelled hydrolysate from algae (*Galdieria sulphuraria*) | 4 |
| ¹³C, ¹⁵N uniformly stable isotope labelled hydrolysate (enzymatic) from methylotrophs (*Methylobacillus flagellatus*) | 7 |
| ¹³C, ¹⁵N uniformly stable isotope labelled yeast autolysate (*Hansenula polymorpha*) | 3 |
| ¹³C uniformly stable isotope labelled glucose | 5 |
| ¹³C, ¹⁵N uniformly stable isotope labelled lipid extract (*Porpyridium cruentum*) | 0.15 |
| Pluronic F-68 | 1 |

Lipid extract was added to the medium as an ethanolic solution that was prepared in a concentration of 150 mg of lipid extract (Example) per 1 ml of ethanol. Ethanol lipid solution was added in a concentration 1 ml/l culture medium. The hydrolysates were analysed by amino acid analysis (RP-HPLC as FMOC derivatives) and deficient amino acids (according to the DMEM-formulation) such as ¹³C, ¹⁵N labelled cysteine and ¹³C, ¹⁵N glutamine were added separately to the medium in a concentration 70 mg/l and 1000 mg/l accordingly. pH was adjusted to 7.2, osmolarity is adjusted to 310 mOsmol kg-1 (NaCl). The complete medium was sterilised through a 0.22 micron filter and stored at 4 °C. With 5-10 passages the CHO cell line was adapted to growth in isotopically labelled medium according to (Bosman, et al. in Method Mol. Biology V. 228, Ed. Selinsky, Humana Press Inc). CHO cells are cultured at a temperature of 37 °C and do require ¹³CO₂ supplementation. Doubling time of adapted cells was 28-30 hours which is comparable to other commercially available serum-free media (C4726, Sigma, Ex-Cell 302, JRH, CD CHO AGT, Invitrogen). Maximum cell density achieved was 4-6 x 10⁶ cells/ml.

### 6.2.2 Composition of stable isotope labelled nutrient medium suitable for HEK 293 cells

Composition of medium with respect to microelements, salts and vitamins was based on the DMEM formulation (see e.g. JRH Biosciences, Cell culture and services), except that carbon and nitrogen containing salts were replaced by stable-isotope labelled ones. Other components of the medium were added in the concentration given in the Table below.

| Components | Grams/Litre |
|---|---|
| ¹³C, ¹⁵N uniformly stable isotope labelled autolysate from algae (*Galdieria sulphuraria*) | 2 |
| ¹³C, ¹⁵N uniformly stable isotope labelled hydrolysate (enzymatic) from methylotrophs (*Methylobacillus flagellatus*) | 4 |
| ¹³C, ¹⁵N uniformly stable isotope labelled yeast autolysate (*Hansenula polymorpha*) | 6 |
| ¹³C uniformly stable isotope labelled glucose | 5 |
| ¹³C, ¹⁵N uniformly stable isotope labelled lipid extract (*Porpyridium cruentum*) | 0.15 |
| Pluronic F-68 | 1 |

Lipid extract was added to the medium as an ethanolic solution that was prepared in a concentration of 150 mg of lipid extract (Example) per 1 ml of ethanol. Ethanol lipid solution was added in a concentration 1 ml/l culture medium. The hydrolysates were analysed by amino acid analysis (RP-HPLC as FMOC derivatives) and deficient amino acids (according to the DMEM-formulation) such was ¹³C, ¹⁵N labelled cysteine and ¹³C, ¹⁵N glutamine were added separately to the medium in a concentration 70 mg/l and 1000 mg/l accordingly. pH was adjusted to 7.2, osmolarity is adjusted to 310 mOsmol kg-1 (NaCl). The complete medium was sterilised through a 0.22 micron filter and stored at 4 °C. With 5-10 passages the HEK-293 cell line was adapted to growth in isotopically labelled medium according to (Bosman, et al. in Method Mol. Biology V. 228, Ed. Selinsky, Humana Press Inc). HEK-293cells are cultured at a temperature of 37 °C and do require ¹³CO₂ supplementation. Doubling time of adapted cells was 26-30 hours which is comparable to other commercially available serum-free media (C4726, Sigma, Ex-Cell 302, JRH, CD CHO AGT, Invitrogen). Maximum cell density achieved was 3-5 x 10⁶ cells/ml.

### 6.2.3 Composition of stable isotope labelled nutrient medium suitable for CHO cells

Composition of medium with respect to microelements, salts and vitamins was based on the DMEM formulation (see e.g. JRH Biosciences, Cell culture and services), except that carbon and nitrogen containing salts were replaced by stable-isotope labelled ones. Other components of the medium were added in the concentration given in the Table below.

| Components | Grams/Litre |
|---|---|
| ¹⁵N uniformly stable isotope labelled hydrolysate from algae (*Scenedesmus obliquus)* | 1 |
| ¹⁵N uniformly stable isotope labelled yeast autolysate (*Hansenula polymorpha*) | 4 |
| Glucose | 5 |
| ¹⁵N uniformly stable isotope labelled lipid extract (*Galdieria sulphuraria*) | 0.15 |
| Pluronic F-68 | 1 |

Lipid extract was added to the medium as an ethanolic solution that was prepared in a concentration of 150 mg of lipid extract (Example) per 1 ml of ethanol. Ethanol lipid solution was added in a concentration 1 ml/l culture medium. The hydrolysates were analysed by amino acid analysis (RP-HPLC as FMOC derivatives) and deficient amino acids (according to the DMEM-formulation) such as ¹⁵N labelled cysteine and ¹⁵N₂ glutamine were added separately to the medium in a concentration 70 mg/l and 1000 mg/l accordingly. pH was adjusted to 7.2, osmolarity is adjusted to 310 mOsmol kg-1 (NaCl). The complete medium was sterilized through a 0.22 micron filter and stored at 4 °C. With 5-10 passages the CHO cell line was adapted to growth in isotopically labelled medium according to (Bosman, et al. in Method Mol. Biology V. 228, Ed. Selinsky, Humana Press Inc). CHO cells are cultured at a temperature of 37 °C and do require CO₂ supplementation. Doubling time of adapted cells was 26-30 hours which is comparable to other commercially available serum-free media (C4726, Sigma, Ex-Cell 302, JRH, CD CHO AGT, Invitrogen). Maximum cell density achieved was 4-6 x 10⁶ cells/ml.

### Example 7. Production of isotopically labelled recombinant protein

### 7.1.1 Isotopically labelled recombinant aquaporin-2 produced in insect cells

Sf9 cells were grown in a 500 ml spinner bottle containing 100 ml of culture medium. Culture medium is formulated as shown in Example 6.1.2. Sf9 cells were obtained from ATCC (CRL-1711) and adapted to a formulated medium by step-wise adaptation as described by Bosman et al. supra. Sf9 cells were inoculated at a density of 3 x 10⁵ cells/ml and were grown to a density of 2 x 10⁶ cells/ml. Then cells were infected with recombinant baculovirus encoding for His tagged aquaporin-2 (AQP2) constructed as described in Werten et.al. (2001, FEBS Lett. 504: 200). Baculovirus was added with a MOI of 1.0. Daily a sample of 10 µl of culture was taken to follow expression of HT-AQP2 by dot-blotting. Expression level was maximal at about 3 days dpi (days post-infection). Cells were harvested by 10 min centrifugation at 5000 g at 4°C. Recombinant HT-AQP2 was purified by urea stripping followed by immobilised metal-affinity chromatography over Ni-NTA beads (Qiagen) as described in Werten et al. (2001, *supra).* Purified Ht-AQP was eluted with 100 mM L- histidine and reconstituted into proteoliposomes by mixing with E. coli lipids and overnight dialysis against buffer containing 20 mM phosphate, 50 mM NaCl, pH 7.2 at 4° C. Finally, an amount of 60 µg of purified protein was obtained. Stable isotope label incorporation was checked by FTIR spectroscopy similar to as described in (Talvenheimo et al., 2002, Biopolymers 67(1): 10-19) and was found to be 98% (¹³C) and 99% (¹⁵N).

### 7.1.2 Isotopically labelled recombinant histamine H1 receptor produced in insect cells

c-DNA encoding a His-tagged humane histamine 1 receptor (Ht-H1) was prepared for expression from recombinant baculovirus in Sf9 cells as described by Ratnala et al. (2004, Eur. J. Biochem. 271: 2636-46). Recombinant virus was purified by plaque assay and amplified (Klaasen and de Grip, 2000, Methods Enzymology, 315: 12-29). Sf9 cells were grown in a 500 ml spinner bottle containing 100 ml of culture medium formulated as shown in the example 6.1.2. Sf9 cells were obtained from ATCC (CRL-1711) and adapted to a formulated medium by step-wise adaptation as described by Bosman et al. (*supra*). Sf9 cells were inoculated at a density of 3 x 10⁵ cells/ml and were grown to a density of 3 x 10⁶ cells/ml. Then cells were infected with recombinant baculovirus encoding the His-tagged histamine H1 receptor (HT-H1) constructed as described above. Baculovirus was added at a MOI of 1.0. Daily samples of 10 µl of culture were taken to follow expression of HT-H1 by dot-blotting. Expression level was maximal at about 4 days dpi (days post-infection). Cells were harvested by 10 min centrifugation at 5000 g at 4°C. Functionality was checked by the radioactive ligand binding assay as described Ratnala et al. (2004, *supra*).

Recombinant HT-H1 was purified by means of immobilised metal-affinity chromatography over Ni-NTA beads (Qiagen, Germany) as described in Ratnala et al. (2004, *supra*). Purified HT-H1 was eluted with 150 mM imidazole and reconstituted into proteoliposomes by mixing with asolectin followed by cyclodextrin extraction of detergent as described by de Grip et al. (1998, Biochem. J. 330: 667-674). Finally, an amount of 250 µg of purified functional protein was obtained.

Stable isotope label incorporation was checked by FTIR spectroscopy (Mattson Cygnus 100 FTIR spectrometer, Madision, WI) by quantitation of the vibrational shifts induced by the stable isotope labelling and was found to be more than 95 % for both nuclei ¹³C ¹⁵N (see below in Example 8).

In addition, 15N SSNMR data were collected on a Bruker 750 spectrometer at 200 K and spinning speed of 8-12 kHz to optimise sampling conditions for the follow up structural studies (see Figure 1). The procedure was subsequently scaled up to a bioreactor (4 liters) with a volumetric yield of at least 4 mg of functional stable isotope labelled HT-H1 receptor per liter.

### 7.1.3.Isotopically labelled recombinant histamine H1R receptor produced in CHO cells

c-DNA encoding the humane histamine 1 receptor was cloned into the pcDEF vector, obtained from Prof. J.Langer, Robert Wood Johnson Medical School, Piscataway, NJ, USA). CHO cells were grown in a 500 ml spinner bottle containing 100 ml of culture medium formulated as shown in the example 6.2.3. CHO cells were adapted to a formulated medium by step-wise adaptation as described by Bosman, et al. supra. CHO cells were inoculated at a density of 6 x 10⁵ cells/ml and were grown to a density of 3 x 10⁶ cells/ml. Then cells were transfected with the plasmid encoding for His- tagged histamine H1 receptor constructed as described above. Daily a sample of 10 µl of culture was taken to follow expression of HT-H1 by dot-blotting. Expression level was maximal at about 3 to 4 days dpt (days post-transfection). Cells were harvested by 10 min centrifugation at 5000 g at 4°C. Functionality was checked by the radioactive ligand binding assay as described (Ratnala et al., *supra*). Recombinant HT-H1 was purified and reconstituted as it is described in the example 7.1.2 and an amount of 150 µg of purified functional protein was obtained. Stable isotope label incorporation was checked by FTIR spectroscopy (Mattson Cygnus 100 FTIR spectrometer, Madision, WI) as described below in Example 8 by quantitating of the vibrational shifts induced by the stable isotope labelling and was found to be more than 95 % for nuclei ( ¹⁵N). The procedure could be scaled up to a bioreactor level with similar volumetric yields of the functional receptor, namely at least 2.5 mg of functional stable isotope labeled receptor per liter.

### Example 8. Fourier Transformed Infra Red spectroscopy for compositional analysis of biomass and for monitoring of stable isotope labelling

### 8.1 Background on FTIR- method

Major biomolecular classes (protein, lipid, carbohydrate) can be easily identified by Fourier Transformed Infra Red spectroscopy (FTIR) since typical biomolecular classes absorb in different frequency ranges: 1500-1700 cm⁻¹ for protein peptide amide groups, 1700-1750 cm⁻¹ and 2800-3000 cm⁻¹ for lipid ester groups and C-H bonds respectively and 1000-1200 cm⁻¹ for carbohydrate C-OH and C-O-C groups. Since the molar absorbance of these vibrational transitions differ significantly, a quantitative estimation of biomass composition cannot be easily achieved, but a qualitative compositional analysis is allowed, that is very useful to compare different biomass batches as is shown in Figure 2.

Vibrational frequency depends on the mass of participating atoms, hence FTIR can be very well applied as well to determine isotope label incorporation. A ¹⁵N label will shift the amide II vibration, of the peptide bond, which absorbs within 1520-1550 cm⁻¹ and is dominated by the CN-H bending vibration, by 10-20 cm⁻¹. A ¹³C label will shift the amide 1 vibration of the peptides bond, that absorbs within 1620-1670 cm⁻¹ and is dominated by the C=O stretch vibration, by 40-60 cm⁻¹, and in addition will shift the C-OH and C-O-C vibrations of carbohydrate moieties by 30-50 cm⁻¹. A ²H label, finally, will shift the C-H vibration of lipid groups by about 700 cm⁻¹ and the amide II vibration of proteins by about 100 cm⁻¹. Using peak separation strategies (deconvolution, second derivative) peak shifts due to label incorporation can be quantitatively estimated with an accuracy of about 5%. Thus this technique enables both: rapid compositional analysis of the biomass and biomass-derived biomolecules, as well as monitoring of stable isotope labelling. Determination of e.g. ¹⁵N labelling efficiency can be achieved by monitoring the absorbance ratio A1542/A1534 and A1518/1534 in the amide II region and is shown in Figure 3. The amide II band, mainly representing the N-H bending vibration within the protein backbone, shifts from about 1542 to 1530 cm-1 upon ¹⁵N labelling. However, the the neighboring amide I band around 1658 cm⁻¹ (mainly C=O stretching vibration) and Tyr residue side-chain vibrational band around 1518 cm⁻¹ are not effected by isotope substitution. The absorption around 1518 cm⁻¹ does actually change upon isotope substitution (downshift of the other amide II to around 1530 cm⁻¹).

### 8.2 FTIR materials and methods

FTIR spectra were taken at ambient temperature on a Mattson Cygnus 100 IR spectrometer, either in the ATR (attenuated total reflection) mode or in the transmission mode, at a resolution of 8 cm⁻¹ and a spectral range of 4000-800 cm⁻¹. For ATR analysis biomass was suspended in deionised water (about 20 mg/ml) in a bath sonicator and a volume containing about 5 mg of biomass was applied evenly over the surface of the Germanium plate of a Specac ATR accessory. After dehydration overnight on air a thin film of biomass had been deposited on the Germanium plate. Subsequently, the ATR accessory was installed in the spectrometer and the biomass film was further dehydrated in the nitrogen gas purge of the spectrometer until water vapor was no longer detectable or by spin-drying (Clark, et al., (1980) Biophys. J. 31, 65-96). For transmission analysis a volume of suspended biomass containing 0,5-1,0 mg was applied to a AgCl window (Fisher Scientific, 1.6 cm diameter) and dehydrated overnight on air. The window was then inserted in a home-made sample-changer brought under computer control, installed in the spectrometer and further dehydrated in the nitrogen gas purge. Finally, spectra were taken after water vapour was no longer detectable. Second derivative spectra were calculated from the absorbance spectra using Mattson software installed in the computer controlling the spectrometer.

### 8.3 Analysis of stable isotope labelled components

Batch-to-batch reproducibility of stable isotope labelled media components was checked via FTIR spectroscopy. Protein content in biomass has been estimated from elemental analysis via nitrogen determination and amino acid analysis after complete hydrolysis using FMOC derivatisation. Creatine phosphokinase from rabbit muscle (EC 2.7.3.2., Boehringer, Mannheim) was used as a standard for the quantitative determination of proteins in biomass preparations using FTIR.

Derivatisation with trinitrobenzensulfonic acid (TNBS) was used to determine total amino group containing components in the hydrolyzates and autolysates. However this TNBS method was not applicable to the hydrolysates neutralised with ¹⁵N ammonium hydroxide due to reaction with the ammonia.

## Claims

1. A method for producing a nutrient medium for growing mammalian or insect cells in culture whereby for at least one of H, C or N, 95% or more of the atoms in substrates that are used by the cells for synthesis of biomolecules in the nutrient medium are isotopically labelled, whereby the method comprising the steps of:
(a) growing an organism on a mineral medium which supports growth of the organism, whereby in the medium 95% or more of the assimilable atoms, for at least one of H, C or N, are isotopically labelled, to produce labelled biomass;
(b) autolysing the biomass of an organism grown as in (a) to produce an autolysate; and,
(c) composing the nutrient medium by combining the autolysate as obtained in (b) with further components necessary for growth of the mammalian or insect cells,
wherein the organism is a fungus, yeast or algae.

2. A method according to claim 1, wherein the organism is an organism that belongs to a genus selected from *Saccharomyces, Pichia, Hansenula, Kluyveromyces, Candida, Brettanomyces, Debaryomyces, Tolrulopsis, Yarrowia, Galdieria, Cyanidium, Porphyridium, Cystoclonium, Audouinella,* and *Cyanidioschyzon.*

3. A method according to claim 1 or 2, wherein the method further comprises the steps of:
(d) growing an organism on a mineral medium which supports growth of the organism, whereby in the medium 95% or more of the assimilable atoms, for at least one of H, C or N, are isotopically labelled, to produce labelled biomass;
(e) extracting biomass of an organism with an organic solvent to produce an extract comprising lipids, whereby the organism is grown as in (d) or is grown as in (d) on a medium without isotopic substitution;
(f) hydrolysing biomass of an organism grown as in (d) at a non-alkaline pH to produce a hydrolysate comprising amino acids;
(g) composing the nutrient medium by combining the autolysate as obtained in any one of claims 1 - 3 with amino acids as obtained in (f) and the lipids obtained in (e) and adding further components necessary for growth of the mammalian or insect cells.

4. A method according to claim 3, whereby the nutrient medium is composed of autolysate, lipids and amino acids obtained from at least two different organisms.

5. A method according to claim 3 or 4, whereby, prior to hydrolysis in (f), lipids and pigments are extracted from the biomass using an organic solvent.

6. A method according to any one of claims 3 - 5, whereby the organism from which the lipids are extracted, belongs to a genus selected from the group consisting of *Rhodophyta, Cyanidiophyceae, Chlorophyta, Cyanophyta, Diatoms, Phaeophyceae, Dinoflagelate, Dinophyta* and *Galdieria.*

7. A method according to any one of claims 3 - 6, whereby the organism from which the hydrolysate comprising amino acids is produced, is an organism selected from the group consisting of algae, fungi, yeasts and methylotrophic bacteria.

8. A method according to claim 7, whereby the organism belongs to a genus selected from the group consisting *of Pichia, Saccharomyces, Hansenula, Cyanidium, Galdieria, Porphyridium, Spirulina,* and *Methylobacillus.*

9. A method according to any one of claims 1 - 8, whereby the further components necessary for growth of the mammalian or insect cells comprise one or more of
(a) one or more of glucose, fructose, and sucrose;
(b) one or more Krebs-cycles intermediates selected from the group consisting of citrate, succinate, fumarate, maleic acid, oxalate and malate;
(c) pyruvate; and,
(d) one or more vitamins selected from the group consisting of thiamin, riboflavin, niacin, vitamin B6, folic acid, vitamin B12, biotin, pantothenic acid, choline, para-aminobenzoic acid and alpha-tocopherol.

10. A method according to any one of claims 1 - 9, whereby 95% or more of the atoms in substrates that are used by the mammalian or insect cells for synthesis of biomolecules in the nutrient medium are isotopically labelled with an isotope selected from ¹⁵N; ¹³C; ²H; ¹⁵N and ¹³C; ¹⁵N and ²H; ¹³C and ²H; or ¹⁵N, ¹³C and ²H.

11. A method for producing a biomolecule, whereby 95% or more of the atoms in the biomolecule are isotopically labelled, the method comprising the steps of:
(a) producing a nutrient medium for growing mammalian or insect cells in culture in a method according to any one of claims 1 - 10;
(b) growing a culture of mammalian or insect cells capable of producing the biomolecule under conditions conducive to the production of the biomolecule in the nutrient medium; and
(c) recovery of the biomolecule.

12. A method according to claim 11, wherein the biomolecule is a soluble protein or a membrane protein.

13. A method according to claim 12, wherein the mammalian or insect cells capable of producing the protein comprise an expression vector comprising a nucleotide sequence coding for the protein.

14. A method according to claims 12 or 13, wherein the protein is a mammalian protein.

15. A method for obtaining structural information on a biomolecule, the method comprising the steps of:
(a) producing a biomolecule, whereby 95% or more of the atoms in the biomolecule are isotopically labelled, in a method according to any one of claims 11 - 14; and,
(b) subjecting the biomolecule to spectroscopic analysis to determine information about its structure.

16. A method according to claim 15, wherein the labelled biomolecule obtained in (a) is purified prior to spectroscopic analysis in step (b).

17. A method according to claim 15 or 16, wherein the spectroscopic analysis comprises NMR spectroscopy.

18. A method according to any one of claims 15 - 17, wherein the structural information on a biomolecule is information about the three-dimensional structure of the biomolecule.

19. A method according to any one of claims 15 - 18, wherein the biomolecule is a protein complexed to a second biomolecule.

20. A method according to claim 19, wherein the second biomolecule is produced in a method according to any one of claims 12 - 15 and whereby 20 - 100% of the hydrogen atoms in the second biomolecule are uniformly substituted with the isotope ²H.

21. A method according to claim 20, wherein the second biomolecule is a protein.

## Patentansprüche

1. Verfahren zur Herstellung eines Nährmediums zum Züchten von Säuger- oder Insektenzellen in Kultur, wobei bei mindestens einem von H, C oder N 95% oder mehr der Atome in den Substraten, die von den Zellen für die Synthese von Biomolekülen im Nährmedium verwendet werden, isotopisch markiert sind, wobei das Verfahren die folgenden Schritte umfasst:
(a) Züchten eines Organismus auf einem mineralischen Medium, das das Wachstum des Organismus unterstützt, wobei im Medium 95% oder mehr der assimilierbaren Atome von mindestens einem von H, C oder N isotopisch markiert sind, um markierte Biomasse herzustellen;
(b) Autolysieren der Biomasse eines in (a) gezüchteten Organismus, um ein Autolysat herzustellen; und
(c) Herstellen des Nährmediums durch Kombinieren des in (b) erhaltenen Autolysats mit weiteren Bestandteilen, die für das Wachstum der Säuger- oder Insektenzellen erforderlich sind,
wobei der Organismus ein Pilz, eine Hefe oder eine Alge ist.

2. Verfahren nach Anspruch 1, wobei der Organismus ein Organismus ist, der zu einer Gattung ausgewählt aus *Saccharomyces, Pichia, Hansenula, Kluyveromyces, Candida, Brettanomyces, Debaryomyces, Tolrulopsis, Yarrowia, Galdieria, Cyanidium, Porphyridium, Cystoclonium, Audouinella* and *Cyanidioschyzon* gehört.

3. Verfahren nach Anspruch 1 oder 2, wobei das Verfahren des Weiteren die folgenden Schritte umfasst:
(d) Züchten eines Organismus auf einem mineralischen Medium, das das Wachstum des Organismus unterstützt, wobei in dem Medium 95% oder mehr der assimilierbaren Atome von mindestens einem von H, C oder N isotopisch markiert sind, um markierte Biomasse herzustellen;
(e) Extrahieren von Biomasse aus einem Organismus mit einem organischen Lösungsmittel, um einen Extrakt herzustellen, der Lipide umfasst, wobei der Organismus wie in (d) gezüchtet wird oder wie in (d) auf einem Medium ohne isotopische Substitution gezüchtet wird;
(f) Hydrolysieren von Biomasse aus einem wie in (d) gezüchteten Organismus bei einem nicht-basischen pH-Wert, um ein Hydrolysat herzustellen, das Aminosäuren umfasst;
(g) Herstellen des Nährmediums durch Kombinieren des in einem der Ansprüche 1 bis 3 erhaltenen Autolysats mit in (f) erhaltenen Aminosäuren und den in (e) erhaltenen Lipiden und Hinzufügen weiterer Bestandteile, die für das Wachstum der Säuger- oder Insketenzellen erforderlich sind.

4. Verfahren nach Anspruch 3, wobei das Nährmedium aus Autolysat, Lipiden und Aminosäuren, die aus mindestens zwei verschiedenen Organismen erhalten wurden, zusammengesetzt ist.

5. Verfahren nach Anspruch 3 oder 4, wobei vor der Hydrolyse in (f) Lipide und Pigmente mit Hilfe eines organischen Lösungsmittels aus der Biomasse extrahiert werden.

6. Verfahren nach einem der Ansprüche 3 bis 5, wobei der Organismus, aus dem die Lipide extrahiert werden, zu einer Gattung gehört, die ausgewählt ist aus der Gruppe bestehend aus *Rhodophyta, Cyanidiophyceae, Chlorophyta, Cyanophyta, Diatomeen, Phaeophyceae, Dinoflagelate, Dinophyta* and *Galdieria.*

7. Verfahren nach einem der Ansprüche 3 bis 6, wobei der Organismus, aus dem das Aminosäuren enthaltende Hydrolysat hergestellt ist, ein Organismus ist, der ausgewählt ist aus der Gruppe bestehend aus Algen, Pilzen, Hefen und methylotrophen Bakterien.

8. Verfahren nach Anspruch 7, wobei der Organismus zu einer Gattung gehört, die ausgewählt ist aus der Gruppe bestehend aus *Pichia, Saccharomyces, Hansenula, Cyanidium, Galdieria, Porphyridium, Spirulina* und *Methylobacillus.*

9. Verfahren nach einem der Ansprüche 1 bis 8, wobei die weiteren Bestandteile, die für das Wachstum der Säuger- oder Insektenzellen erforderlich sind, eines oder mehrere aus den Folgenden umfassen:
(a) eines oder mehrere aus Glucose, Fructose und Saccharose;
(b) eines oder mehrere aus Krebs-Zyklus-Zwischenprodukten, ausgewählt aus der Gruppe bestehend aus Citrat, Succinat, Fumarat, Maleinsäure, Oxalat und Malat;
(c) Pyruvat; und
(d) ein oder mehrere Vitamine ausgewählt aus der Gruppe bestehend aus Thiamin, Riboflavin, Niacin, Vitamin B6, Folsäure, Vitamin B12, Biotin, Pantothensäure, Cholin, para-Aminobenzoesäure und Alpha-Tocopherol.

10. Verfahren nach einem der Ansprüche 1 bis 9, wobei 95% oder mehr der Atome in Substraten, die von den Säuger- oder Insektenzellen für die Synthese von Biomolekülen in dem Nährmedium verwendet werden, mit einem Isotop ausgewählt aus ¹⁵N; ¹³C; ²H; ¹⁵N und ¹³C; ¹⁵N und ²H; ¹³C und ²H; oder ¹⁵N, ¹³C und ²H isotopisch markiert sind.

11. Verfahren zur Herstellung eines Biomoleküls, wobei 95% oder mehr der Atome in dem Biomolekül isotopisch markiert sind, wobei das Verfahren die folgenden Schritte umfasst:
(a) Herstellen eines Nährmediums zum Züchten von Säuger- oder Insektenzellen in Kultur mit einem Verfahren gemäß einem der Ansprüche 1 bis 10;
(b) Wachsenlassen einer Kultur von Säuger- oder Insektenzellen, die fähig sind, das Biomolekül unter Bedingungen herzustellen, die die Produktion des Biomoleküls im Nährmedium fördern; und
(c) Gewinnen des Biomoleküls.

12. Verfahren nach Anspruch 11, wobei das Biomolekül ein lösliches Protein oder ein Membranprotein ist.

13. Verfahren nach Anspruch 12, wobei die Säuger- oder Insektenzellen, die zur Herstellung des Proteins fähig sind, einen Expressionsvektor umfassen, der eine das Protein codierende Nucleotidsequenz umfasst.

14. Verfahren nach Anspruch 12 oder 13, wobei das Protein ein Säugerprotein ist.

15. Verfahren zum Erhalten von Strukturinformation zu einem Biomolekül, wobei das Verfahren die folgenden Schritte umfasst:
(a) Herstellen eines Biomoleküls, wobei 95% oder mehr der Atome in dem Biomolekül isotopisch markiert sind, mit einem Verfahren nach einem der Ansprüche 11 bis 14; und
(b) Durchführen einer spektroskopischen Analyse mit dem Biomolekül, um Information zu seiner Struktur zu ermitteln.

16. Verfahren nach Anspruch 15, wobei das markierte Biomolkül, das in (a) erhalten wurde, vor der spektroskopischen Analyse in Schritt (b) aufgereinigt wird.

17. Verfahren nach Anspruch 15 oder 16, wobei die spektroskopischen Analyse NMR-Spektroskopie umfasst.

18. Verfahren nach einem der Ansprüche 15 bis 17, wobei die Strukturinformation zu einem Biomolekül Information zur dreidimensionalen Struktur des Biomoleküls ist.

19. Verfahren nach einem der Ansprüche 15 bis 18, wobei das Biomolekül ein Protein ist, das mit einem zweiten Biomolekül komplexiert ist.

20. Verfahren nach Anspruch 19, wobei das zweite Biomolekül mit einem Verfahren nach einem der Ansprüche 12 bis 15 hergestellt wird und wobei 20 bis 100% der Wasserstoffatome in dem zweiten Biomolekül einheitlich durch das ²H-Isotop ersetzt sind.

21. Verfahren nach Anspruch 20, wobei das zweite Biomolekül ein Protein ist.

## Revendications

1. Méthode de production d'un milieu nutritif destiné à la croissance en culture de cellules de mammifères ou d'insectes dans laquelle pour au moins un des atomes H, C ou N, 95 % ou plus des atomes dans les substrats utilisés par les cellules pour la synthèse de biomolécules dans le milieu nutritif sont marqués isotopiquement, la méthode comprenant les étapes de :
(a) croissance d'un organisme sur un milieu minéral qui supporte la croissance de l' organisme, dans le milieu de culture duquel, 95 % ou plus des atomes assimilables, parmi au moins un des atomes de H, C ou N, sont isotopiquement marqués, afin de produire une biomasse marquée ;
(b) autolyse de la biomasse d'un organisme cultivé comme en (a) pour produire un autolysat ; et,
(c) composition du milieu nutritif en combinant l'autolysat tel qu'obtenu en (b) avec d'autres composants nécessaires à la croissance des cellules de mammifères ou d'insectes,
dans laquelle l'organisme est un champignon, une levure ou une algue.

2. Méthode selon la revendication 1, dans laquelle l'organisme est un organisme qui appartient à un genre choisi parmi *Saccharomyces, Pichia, Hansenula, Kluyveromyces, Candida, Brettanomyces, Debaryomyces, Tolrulopsis, Yarrowia, Galdieria, Cyanidium, Porphyridium, Cystoclonium, Audouinella* et *Cyanidioschyzon.*

3. Méthode selon la revendication 1 ou 2, dans laquelle la méthode comprend en outre les étapes consistant à :
(d) cultiver un organisme sur un milieu minéral qui permet la croissance de l'organisme, dans le milieu de culture duquel 95 % ou plus des atomes assimilables, parmi au moins un des atomes H, C ou N, sont isotopiquement marqués, afin de produire une biomasse marquée ;
(e) extraire la biomasse d'un organisme avec un solvant organique pour produire un extrait comprenant des lipides, l'organisme étant cultivé comme en (d) ou cultivé comme en (d) sur un milieu sans substitution isotopique ;
(f) hydrolyser la biomasse d'un organisme cultivé comme en (d) à un pH non alcalin pour produire un hydrolysat comprenant des acides aminés ;
(g) composer le milieu nutritif en combinant l'autolysat tel qu'obtenu dans l'une quelconque des revendications 1 à 3 avec des acides aminés tels qu' obtenus en (f) et les lipides obtenus en (e) et en ajoutant d'autres composants nécessaires à la croissance des cellules de mammifères ou d'insectes.

4. Méthode selon la revendication 3, le milieu nutritif étant composé d'un autolysat, de lipides et d'acides aminés obtenus à partir d'au moins deux organismes différents.

5. Méthode selon la revendication 3 ou 4, dans laquelle, avant l'hydrolyse en (f), des lipides et des pigments sont extraits à partir de la biomasse en utilisant un solvant organique.

6. Méthode telle que revendiquée dans l'une quelconque des revendications 3 à 5, dans laquelle l'organisme à partir duquel les lipides sont extraits, appartient à un genre choisi parmi le groupe constitué des *Rhodophyta, Cyanidiophyceae, Chlorophyta, Cyanophyta*, Diatomées, *Phaephyceae*, Dinoflagellés, *Dinophyta* et *Galdieria.*

7. Méthode selon l'une quelconque des revendications 3 à 6, dans laquelle l'organisme à partir duquel l'hydrolysat comprenant des acides aminés est produit, est un organisme choisi parmi le groupe constitué d'algues, de champignons, de levures et de bactéries méthylotrophes.

8. Méthode selon la revendication 7, dans laquelle l'organisme appartient à un genre choisi parmi le groupe constitué de *Pichia, Saccharomyces, Hansenula, Cyanidium, Galdieria, Porphyridium, Spirulina,* et *Methylobacillus.*

9. Méthode selon l'une quelconque des revendications 1 à 8, les composants nécessaires à la croissance des cellules de mammifères ou d'insectes comprenant un ou plusieurs des composants suivants :
(a) un ou plusieurs sucres parmi le glucose, le fructose et le saccharose ;
(b) un ou plusieurs intermédiaires du cycle de Krebs choisis parmi le groupe constitué du citrate, du succinate, du fumarate, de l'acide maléique, de l'oxalate et du malate ;
(c) le pyruvate ; et,
(d) une ou plusieurs vitamines choisies dans le groupe constitué de la thiamine, la riboflavine, la niacine, la vitamine B6, l'acide folique, la vitamine B12, la biotine, l'acide pantothénique, la choline, l'acide para-aminobenzoïque et l'alphatocophérol.

10. Méthode selon l'une quelconque des revendications 1 à 9, dans laquelle 95 % ou plus des atomes dans les substrats qui sont utilisés par les cellules de mammifères ou d'insectes pour la synthèse de biomolécules dans le milieu nutritif sont marqués isotopiquement avec un isotope choisi parmi ¹⁵N ; ¹³C ; ²H ; ¹⁵N et ¹³C ; ¹⁵N et ²H ; ¹³C et ²H ; ou ¹⁵N, ¹³C et ²H.

11. Méthode de production d'une biomolécule, dans laquelle 95 % ou plus des atomes dans la biomolécule sont isotopiquement marqués, la méthode comprenant les étapes consistant à :
(a) produire un milieu nutritif pour la culture de cellules de mammifères ou d' insectes dans une méthode selon l'une quelconque des revendications 1 à 10 ;
(b) cultiver des cellules de mammifères ou d'insectes capables de produire la biomolécule dans des conditions permettant la production de la biomolécule dans le milieu de culture ; et
(c) récupérer la biomolécule.

12. Méthode selon la revendication 11, dans laquelle la biomolécule est une protéine soluble ou une protéine membranaire.

13. Méthode selon la revendication 12, dans laquelle les cellules de mammifères ou d'insectes capables de produire la protéine comprennent un vecteur d'expression comprenant une séquence nucléotidique codant pour la protéine.

14. Méthode selon les revendications 12 ou 13, dans laquelle la protéine est une protéine de mammifères.

15. Méthode destinée à obtenir une information structurale sur une biomolécule, la méthode comprenant les étapes consistant à :
(a) produire une biomolécule, 95 % ou plus des atomes de la biomolécule étant isotopiquement marqués, dans une méthode selon l'une quelconque des revendications 11 à 14 ; et,
(b) soumettre la biomolécule à une analyse spectroscopique pour déterminer l'information relative à sa structure.

16. Méthode selon la revendication 15, dans laquelle la biomolécule marquée obtenue en (a) est purifiée avant l'analyse spectroscopique à l'étape (b).

17. Méthode selon la revendication 15 ou 16, dans laquelle l'analyse spectroscopique comprend une spectroscopie RMN.

18. Méthode selon l'une quelconque des revendications 15 à 17, dans laquelle l'information structurale sur une biomolécule est une information sur la structure tridimensionnelle de la biomolécule.

19. Méthode selon l'une quelconque des revendications 15 à 18, dans laquelle la biomolécule est une protéine complexée à une seconde biomolécule.

20. Méthode selon la revendication 19, dans laquelle la seconde biomolécule est produite dans une méthode selon l'une quelconque des revendications 12 à 15 et ce par quoi 20 à 100 % des atomes d'hydrogène dans la seconde biomolécule sont uniformément substitués par l'isotope ²H.

21. Méthode selon la revendication 20, dans laquelle la seconde biomolécule est une protéine.
